(19) Europäisches Patentamt
European Patent Office
Office européen des brevets

(11) EP 4 598 922 B1

(12) EUROPEAN PATENT SPECIFICATION

(45) Date of publication and mention of the grant of the patent:
**29.04.2026 Bulletin 2026/18**

(21) Application number: **23783830.5**

(22) Date of filing: **03.10.2023**

(51) International Patent Classification (IPC):
***C07D 471/04*** (2006.01) ***A61P 35/00*** (2006.01)
***A61K 31/437*** (2006.01)

(52) Cooperative Patent Classification (CPC):
**C07D 471/04; A61P 35/00**

(86) International application number:
**PCT/EP2023/077341**

(87) International publication number:
**WO 2024/074497 (11.04.2024 Gazette 2024/15)**

# (54) PARG INHIBITORY COMPOUND

PARG-HEMMENDE VERBINDUNGEN

COMPOSÉS INHIBITEURS PARG

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **03.10.2022 PCT/EP2022/077470**

(43) Date of publication of application:
**13.08.2025 Bulletin 2025/33**

(73) Proprietor: **FoRx Therapeutics AG**
**4056 Basel (CH)**

(72) Inventors:
- **LUECKING, Ulrich**
  **10317 Berlin (DE)**
- **QUEROLLE, Olivier**
  **68300 Saint Louis (FR)**
- **GOUTOPOULOS, Andreas**
  **Boston, Massachusetts 02116 (US)**
- **TIAN, Jin**
  **WuHan East Lake High-tech Development Zone Hubei,**
  **Hubei 430075 (CN)**
- **SOTIRIOU, Sotirios**
  **4056 Basel (CH)**
- **IACOVINO, Luca**
  **4310 Rheinfelden (CH)**
- **FREUDENMANN, Alena**
  **4125 Riehen (DE)**

(74) Representative: **Vossius & Partner**
**Patentanwälte Rechtsanwälte mbB**
**Siebertstraße 3**
**81675 München (DE)**

(56) References cited:
**WO-A1-2016/092326 WO-A1-2021/055744**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 598 922 B1

## Description

### Field of the invention

The present invention relates to a compound of formula (I):

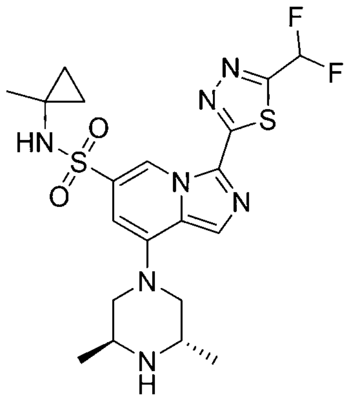

(I)

or pharmaceutically acceptable salt thereof. The present invention further relates to the compound of formula (I) of the present invention for use in therapy. Instant compound is particularly useful as PARG inhibitor and can be used in a method of treatment of a proliferative disorder, preferably of cancer.

### Background of the invention

Cancer is a leading cause of death worldwide. Although progression-free survival and overall survival of cancer patients has improved over the past two decades, millions of cancer patients still have few therapeutic options and poor survival outcomes (Jemal et al., J. Natl. Cancer Inst. 2017, 109, 1975).

DNA replication stress (DRS) is a hallmark of cancer cells and a major source of genomic instability (a) Halazonetis et al., Science 2008, 319, 1352; b) Negrini et al., Nat. Rev. Mol. Cell Biol. 2010, 11, 220). In broad terms, DRS refers to the deregulation of DNA replication and cell cycle progression. DRS can be induced from endogenous or exogenous causes such as oncogene activation and chemotherapeutics, respectively (Zeman and Cimprich, Nat. Cell Biol. 2013, 16, 2). At the level of the replication fork, DRS leads to replication fork stalling, disengagement of the replisome and eventually collapse. Several DNA repair proteins are involved in replication fork stability, protection, and restart under DRS conditions (a) Costantino et al., Science 2014, 343, 88; b) Scully et al., Curr. Opin. Genet. Dev. 2021 71, 154).

Poly(ADP)ribosylation (PARylation) is a transient and reversible post-translational modification that occurs at DNA damaged sites and is catalyzed by the poly (ADP-ribose) polymerase (PARP) family of proteins (Cohen and Chang, Nat. Chem. Biol. 2018, 14, 236). PARylation of various DNA repair proteins leads to their activation. Degradation of the poly(ADP) ribose chains is mediated primarily by the poly(ADP-ribose) glycohydrolase (PARG) protein. DNA damage dependent PARylation/dePARylation is a rapid and dynamic process which needs to be well regulated since imbalances between the two processes can lead to DNA damage.

Human PARG encodes a 111 kDa protein of 976 amino acids. It contains a N-terminal regulatory domain, a catalytic domain and an ADP-ribose binding macrodomain. Five human PARG transcripts have been identified. Full length PARG is mostly nuclear; the smaller isoforms localize primarily to the cytoplasm. PARG functions primarily as an exo-hydrolase and it releases mainly mono(ADP-ribose) by hydrolyzing the $\alpha$-O-glycosidic ribose-ribose bond in PAR. PARG can also act as an endo-hydrolase. PARG preferentially degrades long and linear PAR chains whereas its activity with small and branched PAR chains is significantly reduced (O'Sullivan et al., Nat. Commun. 2019, 10, 1182).

Although PARG is the dominant cellular PAR degrading enzyme, it cannot act on the terminal protein-ribose bond. Additional hydrolases such as terminal ADP-ribose protein glycohydrolase (TARG1) and ADP-ribosylhydrolase 3 (ARH3) are also known to catalyze PAR-degradation. TARG1 and ARH3 complete the reversal of PARylation by removing protein-bound mono(ADP-ribose) moieties (a) Fontana et al., Elife 2017, doi: 10.7554/eLife.28533; b) Rack et al., Genes Dev. 2020, 34, 263). TARG1 is located in the nucleus and cytoplasm. ARH3 is found primarily in the cytoplasm but it can also be found in the mitochondria and in the nucleus (Rack et al., Genes Dev. 2020, 34, 263).

Genomic aberrations targeting tumor suppressor genes or oncogenes, often make cancer cells dependent on specific DNA repair pathways. For instance, it is well known that PARP inhibitors are particularly effective against tumors

carrying mutations in the BRCA1 and BRCA2 genes (a) Bryant et al., Nature 2005, 434, 913; b) Farmer et al., Nature 2005, 434, 917). Targeting synthetic lethal interactions like the one between PARP and BRCA is an attractive novel therapeutic approach for cancer treatment.

PARG participates in DNA replication and in various DNA repair mechanisms including single-strand break (SSB) repair and replication fork restart. PARG inhibitors have shown synthetic lethal phenotype in cells with high levels of DRS caused by low expression of genes involved in DNA replication and/or replication fork stability (Pillay et al., Cancer Cell. 2019, 35, 519). Moreover, PARG inactivation, depletion or inhibition sensitizes cells to irradiation and to DNA damaging agents such as alkylating agents (e.g. temozolomide and methyl methanesulfonate) (a) Fujihara et al., Curr. Cancer Drug Targets 2009, 9, 953; b) Gogola et al., Cancer Cell 2018, 33, 1078; c) Houl et al., Nat Commun. 2019, 10, 5654).

Given the therapeutic potential of PARG inhibitors in cancer treatment, there is an increased need for the development of highly potent and selective PARG inhibitors beyond the ones that have already been described (a) James et al., ACS Chem. Biol. 2016, 11, 3179; b) Waszkowycz et al., J. Med. Chem. 2018, 61, 10767).

Certain compounds that are useful as PARG inhibitors are further disclosed in documents WO 2016/092326, WO 2016/097749 and WO 2021/055744.

Document US 2019/233411 discloses certain Gen2 inhibitors and uses thereof.

Document WO 2009/050183 discloses certain imidazo[1,2-a]pyridine derivatives which are useful for treating diseases mediated by the ALK-5 and/or ALK-4 receptor.

Summary of the invention

It was an objective technical problem of the present invention to provide compounds that are cell-permeable inhibitors of PARG. The technical problem of the present invention is solved by the embodiments described herein and as characterized by the claims. Any references in the description to methods of treatment refer to the compounds, pharmaceutical compositions and medicaments of the present invention for use in a method for treatment of the human (or animal) body by therapy (or for diagnosis).

Accordingly, in a first embodiment, the present invention provides a compound of formula (I):

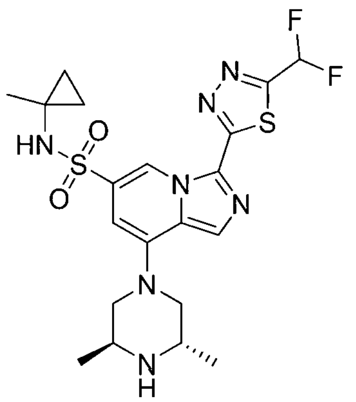

(I)

or tautomer, pharmaceutically acceptable solvate, pharmaceutically acceptable crystal form, or pharmaceutically acceptable salt thereof.

Preferably, in this first embodiment, the present invention provides a compound of formula (I) or a pharmaceutically acceptable salt thereof.

A further embodiment of the present invention relates to a pharmaceutical composition comprising the compound of formula (I) or a pharmaceutically acceptable salt, hydrate or solvate thereof, and a pharmaceutically acceptable carrier.

In a further embodiment, the present invention relates to the compound of formula (I) of the present invention or a pharmaceutically acceptable salt, hydrate or solvate thereof, or a pharmaceutical composition of the present invention, for use in therapy.

The compound of formula (I) is useful for treating a disease or disorder in which PARG activity is implicated.

The compound of formula (I) is useful for a method of treating a proliferative disorder. In a preferred embodiment of the present invention, the proliferative disorder is cancer, preferably a human cancer.

## Definitions

The following definitions apply throughout the present specification and the claims, unless specifically indicated otherwise.

The term "hydrogen" is herein used to refer to protium, deuterium and/or tritium, preferably to protium. Accordingly, the term "non-hydrogen atom" refers to any atoms that is not hydrogen, i.e. that is not protium, deuterium or tritium.

The term "hydrocarbon group" refers to a group consisting of carbon atoms and hydrogen atoms.

The term "alicyclic" is used in connection with cyclic groups and denotes that the corresponding cyclic group is non-aromatic.

As used herein, the term "alkyl" refers to a monovalent saturated acyclic (i.e., non-cyclic) hydrocarbon group which may be linear or branched. Accordingly, an "alkyl" group does not comprise any carbon-to-carbon double bond or any carbon-to-carbon triple bond. A "$C_{1-5}$ alkyl" denotes an alkyl group having 1 to 5 carbon atoms. Preferred exemplary alkyl groups are methyl, ethyl, propyl (e.g., n-propyl or isopropyl), or butyl (e.g., n-butyl, isobutyl, sec-butyl, or tert-butyl). Unless defined otherwise, the term "alkyl" preferably refers to $C_{1-4}$ alkyl, more preferably to methyl or ethyl, and even more preferably to methyl.

As used herein, the term "carbocyclyl" refers to a hydrocarbon ring group, including monocyclic rings as well as bridged ring, spiro ring and/or fused ring systems (which may be composed, e.g., of two or three rings), wherein said ring group may be saturated, partially unsaturated (i.e., unsaturated but not aromatic) or aromatic. Unless defined otherwise, "carbocyclyl" preferably refers to aryl, cycloalkyl or cycloalkenyl.

As used herein, the term "heterocyclyl" refers to a ring group, including monocyclic rings as well as bridged ring, spiro ring and/or fused ring systems (which may be composed, e.g., of two or three rings), wherein said ring group comprises one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, wherein one or more carbon ring atoms may optionally be oxidized (i.e., to form an oxo group), and further wherein said ring group may be saturated, partially unsaturated (i.e., unsaturated but not aromatic) or aromatic. For example, each heteroatom-containing ring comprised in said ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. Unless defined otherwise, "heterocyclyl" preferably refers to heteroaryl, heterocycloalkyl or heterocycloalkenyl.

As used herein, the term "aryl" refers to an aromatic hydrocarbon ring group, including monocyclic aromatic rings as well as bridged ring and/or fused ring systems containing at least one aromatic ring (e.g., ring systems composed of two or three fused rings, wherein at least one of these fused rings is aromatic; or bridged ring systems composed of two or three rings, wherein at least one of these bridged rings is aromatic). "Aryl" may, e.g., refer to phenyl, naphthyl, dialinyl (i.e., 1,2-dihydronaphthyl), tetralinyl (i.e., 1,2,3,4-tetrahydronaphthyl), indanyl, indenyl (e.g., 1H-indenyl), anthracenyl, phenanthrenyl, 9H-fluorenyl, or azulenyl. Unless defined otherwise, an "aryl" preferably has 6 to 14 ring atoms, more preferably 6 to 10 ring atoms, even more preferably refers to phenyl or naphthyl, and most preferably refers to phenyl.

As used herein, the term "heteroaryl" refers to an aromatic ring group, including monocyclic aromatic rings as well as bridged ring and/or fused ring systems containing at least one aromatic ring (e.g., ring systems composed of two or three fused rings, wherein at least one of these fused rings is aromatic; or bridged ring systems composed of two or three rings, wherein at least one of these bridged rings is aromatic), wherein said aromatic ring group comprises one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, and further wherein one or more carbon ring atoms may optionally be oxidized (i.e., to form an oxo group). For example, each heteroatom-containing ring comprised in said aromatic ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heteroaryl" may, e.g., refer to thienyl (i.e., thiophenyl), benzo[b]thienyl, naphtho[2,3-b]thienyl, thianthrenyl, furyl (i.e., furanyl), benzofuranyl, isobenzofuranyl, chromanyl, chromenyl (e.g., 2H-1-benzopyranyl or 4H-1-benzopyranyl), isochromenyl (e.g., 1H-2-benzopyranyl), chromonyl, xanthenyl, phenoxathiinyl, pyrrolyl (e.g., 1H-pyrrolyl), imidazolyl, pyrazolyl, pyridyl (i.e., pyridinyl; e.g., 2-pyridyl, 3-pyridyl, or 4-pyridyl), pyrazinyl, pyrimidinyl, pyridazinyl, indolyl (e.g., 3H-indolyl), isoindolyl, indazolyl, indolizinyl, purinyl, quinolyl, isoquinolyl, phthalazinyl, naphthyridinyl, quinoxalinyl, cinnolinyl, pteridinyl, carbazolyl, β-carbolinyl, phenanthridinyl, acridinyl, perimidinyl, phenanthrolinyl (e.g., [1,10]phenanthrolinyl, [1,7]phenanthrolinyl, or [4,7]phenanthrolinyl), phenazinyl, thiazolyl, isothiazolyl, phenothiazinyl, oxazolyl, isoxazolyl, oxadiazolyl (e.g., 1,2,4-oxadiazolyl, 1,2,5-oxadiazolyl (i.e., furazanyl), or 1,3,4-oxadiazolyl), thiadiazolyl (e.g.,

1,2,4-thiadiazolyl, 1,2,5-thiadiazolyl, or 1,3,4-thiadiazolyl), phenoxazinyl, pyrazolo[1,5-a]pyrimidinyl (e.g., pyrazolo[1,5-a]pyrimidin-3-yl), 1,2-benzoisoxazol-3-yl, benzothiazolyl, benzothiadiazolyl, benzoxazolyl, benzisoxazolyl, benzimidazolyl, benzo[b]thiophenyl (i.e., benzothienyl), triazolyl (e.g., 1H-1,2,3-triazolyl, 2H-1,2,3-triazolyl, 1H-1,2,4-triazolyl, or 4H-1,2,4-triazolyl), benzotriazolyl, 1H-tetrazolyl, 2H-tetrazolyl, triazinyl (e.g., 1,2,3-triazinyl, 1,2,4-triazinyl, or 1,3,5-triazinyl), furo[2,3-c]pyridinyl, dihydrofuropyridinyl (e.g., 2,3-dihydrofuro[2,3-c]pyridinyl or 1,3-dihydrofuro[3,4-c]pyridinyl), imidazopyridinyl (e.g., imidazo[1,2-a]pyridinyl or imidazo[3,2-a]pyridinyl), quinazolinyl, thienopyridinyl, tetrahydrothienopyridinyl (e.g., 4,5,6,7-tetrahydrothieno[3,2-c]pyridinyl), dibenzofuranyl, 1,3-benzodioxolyl, benzodioxanyl (e.g., 1,3-benzodioxanyl or 1,4-benzodioxanyl), or coumarinyl. Unless defined otherwise, the term "heteroaryl" preferably refers to a 5 to 14 membered (more preferably 5 to 10 membered) monocyclic ring or fused ring system comprising one or more (e.g., one, two, three or four) ring heteroatoms independently selected from O, S and N, wherein one or more S ring atoms (if present) and/or one or more N ring atoms (if present) are optionally oxidized, and wherein one or more carbon ring atoms are optionally oxidized; even more preferably, a "heteroaryl" refers to a 5 or 6 membered monocyclic ring comprising one or more (e.g., one, two or three) ring heteroatoms independently selected from O, S and N, wherein one or more S ring atoms (if present) and/or one or more N ring atoms (if present) are optionally oxidized, and wherein one or more carbon ring atoms are optionally oxidized.

As used herein, the term "cycloalkyl" refers to a saturated hydrocarbon ring group, including monocyclic rings as well as bridged ring, spiro ring and/or fused ring systems (which may be composed, e.g., of two or three rings; such as, e.g., a fused ring system composed of two or three fused rings). "Cycloalkyl" may, e.g., refer to cyclopropyl, cyclobutyl, cyclopentyl, cyclohexyl, cycloheptyl, decalinyl (i.e., decahydronaphthyl), or adamantyl. Unless defined otherwise, "cycloalkyl" preferably refers to a $C_{3-11}$ cycloalkyl, and more preferably refers to a $C_{3-7}$ cycloalkyl. A particularly preferred "cycloalkyl" is a monocyclic saturated hydrocarbon ring having 3 to 7 ring members (e.g., cyclopropyl or cyclohexyl).

As used herein, the term "cycloalkenyl" refers to an unsaturated alicyclic (non-aromatic) hydrocarbon ring group, including monocyclic rings as well as bridged ring, spiro ring and/or fused ring systems (which may be composed, e.g., of two or three rings; such as, e.g., a fused ring system composed of two or three fused rings), wherein said hydrocarbon ring group comprises one or more (e.g., one or two) carbon-to-carbon double bonds and does not comprise any carbon-to-carbon triple bond. "Cycloalkenyl" may, e.g., refer to cyclopropenyl, cyclobutenyl, cyclopentenyl, cyclohexenyl, cyclohexadienyl, cycloheptenyl, or cycloheptadienyl. Unless defined otherwise, "cycloalkenyl" preferably refers to a $C_{3-11}$ cycloalkenyl, and more preferably refers to a $C_{3-7}$ cycloalkenyl. A particularly preferred "cycloalkenyl" is a monocyclic unsaturated alicyclic hydrocarbon ring having 3 to 7 ring members and containing one or more (e.g., one or two; preferably one) carbon-to-carbon double bonds.

As used herein, the term "heterocycloalkyl" refers to a saturated ring group, including monocyclic rings as well as bridged ring, spiro ring and/or fused ring systems (which may be composed, e.g., of two or three rings; such as, e.g., a fused ring system composed of two or three fused rings), wherein said ring group contains one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, and further wherein one or more carbon ring atoms may optionally be oxidized (i.e., to form an oxo group). For example, each heteroatom-containing ring comprised in said saturated ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heterocycloalkyl" may, e.g., refer to aziridinyl, azetidinyl, pyrrolidinyl, imidazolidinyl, pyrazolidinyl, piperidinyl, piperazinyl, azepanyl, diazepanyl (e.g., 1,4-diazepanyl), oxazolidinyl, isoxazolidinyl, thiazolidinyl, isothiazolidinyl, morpholinyl (e.g., morpholin-4-yl), thiomorpholinyl (e.g., thiomorpholin-4-yl), oxazepanyl, oxiranyl, oxetanyl, tetrahydrofuranyl, 1,3-dioxolanyl, tetrahydropyranyl, 1,4-dioxanyl, oxepanyl, thiiranyl, thietanyl, tetrahydrothiophenyl (i.e., thiolanyl), 1,3-dithiolanyl, thianyl, 1,1-dioxothianyl, thiepanyl, decahydroquinolinyl, decahydroisoquinolinyl, or 2-oxa-5-aza-bicyclo[2.2.1]hept-5-yl. Unless defined otherwise, "heterocycloalkyl" preferably refers to a 3 to 11 membered saturated ring group, which is a monocyclic ring or a fused ring system (e.g., a fused ring system composed of two fused rings), wherein said ring group contains one or more (e.g., one, two, three, or four) ring heteroatoms independently selected from O, S and N, wherein one or more S ring atoms (if present) and/or one or more N ring atoms (if present) are optionally oxidized, and wherein one or more carbon ring atoms are optionally oxidized; more preferably, "heterocycloalkyl" refers to a 5 to 7 membered saturated monocyclic ring group containing one or more (e.g., one, two, or three) ring heteroatoms independently selected from O, S and N, wherein one or more S ring atoms (if present) and/or one or more N ring atoms (if present) are optionally oxidized, and wherein one or more carbon ring atoms are optionally oxidized.

As used herein, the term "heterocycloalkenyl" refers to an unsaturated alicyclic (non-aromatic) ring group, including monocyclic rings as well as bridged ring, spiro ring and/or fused ring systems (which may be composed, e.g., of two or three rings; such as, e.g., a fused ring system composed of two or three fused rings), wherein said ring group contains one or more (such as, e.g., one, two, three, or four) ring heteroatoms independently selected from O, S and N, and the remaining ring atoms are carbon atoms, wherein one or more S ring atoms (if present) and/or one or more N ring atoms

(if present) may optionally be oxidized, wherein one or more carbon ring atoms may optionally be oxidized (i.e., to form an oxo group), and further wherein said ring group comprises at least one double bond between adjacent ring atoms and does not comprise any triple bond between adjacent ring atoms. For example, each heteroatom-containing ring comprised in said unsaturated alicyclic ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heterocycloalkenyl" may, e.g., refer to imidazolinyl (e.g., 2-imidazolinyl (i.e., 4,5-dihydro-1H-imidazolyl), 3-imidazolinyl, or 4-imidazolinyl), tetrahydropyridinyl (e.g., 1,2,3,6-tetrahydropyridinyl), dihydropyridinyl (e.g., 1,2-dihydropyridinyl or 2,3-dihydropyridinyl), pyranyl (e.g., 2H-pyranyl or 4H-pyranyl), thiopyranyl (e.g., 2H-thiopyranyl or 4H-thiopyranyl), dihydropyranyl, dihydrofuranyl, dihydropyrazolyl, dihydropyrazinyl, dihydroisoindolyl, octahydroquinolinyl (e.g., 1,2,3,4,4a,5,6,7-octahydroquinolinyl), or octahydroisoquinolinyl (e.g., 1,2,3,4,5,6,7,8-octahydroisoquinolinyl). Unless defined otherwise, "heterocycloalkenyl" preferably refers to a 3 to 11 membered unsaturated alicyclic ring group, which is a monocyclic ring or a fused ring system (e.g., a fused ring system composed of two fused rings), wherein said ring group contains one or more (e.g., one, two, three, or four) ring heteroatoms independently selected from O, S and N, wherein one or more S ring atoms (if present) and/or one or more N ring atoms (if present) are optionally oxidized, wherein one or more carbon ring atoms are optionally oxidized, and wherein said ring group comprises at least one double bond between adjacent ring atoms and does not comprise any triple bond between adjacent ring atoms; more preferably, "heterocycloalkenyl" refers to a 5 to 7 membered monocyclic unsaturated non-aromatic ring group containing one or more (e.g., one, two, or three) ring heteroatoms independently selected from O, S and N, wherein one or more S ring atoms (if present) and/or one or more N ring atoms (if present) are optionally oxidized, wherein one or more carbon ring atoms are optionally oxidized, and wherein said ring group comprises at least one double bond between adjacent ring atoms and does not comprise any triple bond between adjacent ring atoms.

As used herein, the term "halogen" refers to fluoro (-F), chloro (-Cl), bromo (-Br), or iodo (-I).

As used herein, the term "haloalkyl" refers to an alkyl group substituted with one or more (preferably 1 to 6, more preferably 1 to 3) halogen atoms which are selected independently from fluoro, chloro, bromo and iodo, and are preferably all fluoro atoms. It will be understood that the maximum number of halogen atoms is limited by the number of available attachment sites and, thus, depends on the number of carbon atoms comprised in the alkyl moiety of the haloalkyl group. "Haloalkyl" may, e.g., refer to $-CF_3$, $-CHF_2$, $-CH_2F$, $-CF_2-CH_3$, $-CH_2-CF_3$, $-CH_2-CHF_2$, $-CH_2-CF_2-CH_3$, $-CH_2-CF_2-CF_3$, or $-CH(CF_3)_2$. A particularly preferred "haloalkyl" group is $-CF_3$.

The terms "bond" and "covalent bond" are used herein synonymously, unless explicitly indicated otherwise or contradicted by context.

As used herein, unless explicitly indicated otherwise or contradicted by context, the terms "a", "an" and "the" are used interchangeably with "one or more" and "at least one". Thus, for example, a composition comprising "a" compound of formula (I) can be interpreted as referring to a composition comprising "one or more" compounds of formula (I).

It is to be understood that wherever numerical ranges are provided/disclosed herein, all values and subranges encompassed by the respective numerical range are meant to be encompassed within the scope of the invention. Accordingly, the present invention specifically and individually relates to each value that falls within a numerical range disclosed herein, as well as each subrange encompassed by a numerical range disclosed herein.

As used herein, the term "about" preferably refers to ±10% of the indicated numerical value, more preferably to ±5% of the indicated numerical value, and in particular to the exact numerical value indicated. If the term "about" is used in connection with the endpoints of a range, it preferably refers to the range from the lower endpoint -10% of its indicated numerical value to the upper endpoint +10% of its indicated numerical value, more preferably to the range from of the lower endpoint -5% to the upper endpoint +5%, and even more preferably to the range defined by the exact numerical values of the lower endpoint and the upper endpoint.

As used herein, the term "comprising" (or "comprise", "comprises", "contain", "contains", or "containing"), unless explicitly indicated otherwise or contradicted by context, has the meaning of "containing, inter alia", i.e., "containing, among further optional elements, ...". In addition thereto, this term also includes the narrower meanings of "consisting essentially of" and "consisting of". For example, the term "A comprising B and C" has the meaning of "A containing, inter alia, B and C", wherein A may contain further optional elements (e.g., "A containing B, C and D" would also be encompassed), but this term also includes the meaning of "A consisting essentially of B and C" and the meaning of "A consisting of B and C" (i.e., no other components than B and C are comprised in A).

## Brief description of Figures

The invention is illustrated using the appended Figures, which serve only an illustrative purpose and should not be interpreted as limiting to the scope of the invention in any way.

**Figure 1** presents the results of testing the compound of Example 1 in MDA-MB-436 xenograft model.

## Detailed description of the invention

The invention is described in detail in the following. It is to be understood that the present invention specifically relates to each and every combination of features and embodiments described herein, including any combination of general and/or preferred features/embodiments.

In a first embodiment, the present invention relates to a compound of formula (I):

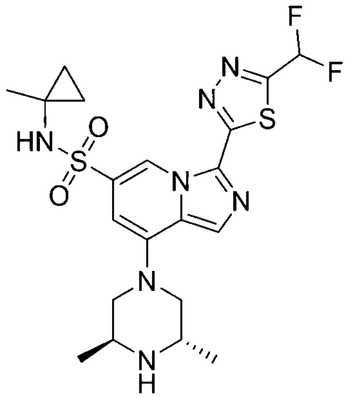

(I)

or a tautomer, pharmaceutically acceptable solvate, pharmaceutically acceptable crystal form, or pharmaceutically acceptable salt thereof. The compound may also be referred to as 3-(5-(difluoromethyl)-1,3,4-thiadiazol-2-yl)-8-((3S,5S)-3,5-dimethylpiperazin-1-yl)-N-(1-methylcyclopropyl)imidazo[1,5-a]pyridine-6-sulfonamide.

As it is to be understood herein, a reference to a tautomer, pharmaceutically acceptable solvate, pharmaceutically acceptable crystal form, or pharmaceutically acceptable salt of a compound is preferably a reference to a pharmaceutically acceptable solvate (such as hydrate), pharmaceutically acceptable crystal form or a pharmaceutically acceptable salt thereof, more preferably to a pharmaceutically acceptable salt thereof.

Preferably, the present invention relates to a compound of formula (I) or a pharmaceutically acceptable salt thereof.

The present invention further specifically relates to a non-salt form of the compound of formula (I). Said non-salt form may also be referred to as a free-base form. Accordingly, within the present invention the compound of formula (I) or a pharmaceutically acceptable salt thereof may be a free base form of the compound of formula (I).

In one embodiment, the present application relates to a formate salt of the compound of formula (I). Accordingly, within the present invention the compound of formula (I) or a pharmaceutically acceptable salt thereof may be a formate salt of the compound of formula (I).

The present invention also relates to each of the intermediates described further below in the examples section of this specification, including any one of these intermediates in non-salt form or in the form of a salt (e.g., a pharmaceutically acceptable salt) of the respective compound. Such intermediates can be used, in particular, in the synthesis of the compound of formula (I).

The scope of the invention embraces all pharmaceutically acceptable salt forms of the compound of formula (I) which may be formed, e.g., by protonation of an atom carrying an electron lone pair which is susceptible to protonation, such as an amino group, with an inorganic or organic acid, or as a salt of an acid group (such as a carboxylic acid group) with a physiologically acceptable cation. Exemplary base addition salts comprise, for example: alkali metal salts such as sodium or potassium salts; alkaline earth metal salts such as calcium or magnesium salts; zinc salts; ammonium salts; aliphatic amine salts such as trimethylamine, triethylamine, dicyclohexylamine, ethanolamine, diethanolamine, triethanolamine, procaine salts, meglumine salts, ethylenediamine salts, or choline salts; aralkyl amine salts such as N,N-dibenzylethylenediamine salts, benzathine salts, benethamine salts; heterocyclic aromatic amine salts such as pyridine salts, picoline salts, quinoline salts or isoquinoline salts; quaternary ammonium salts such as tetramethylammonium salts, tetraethylammonium salts, benzyltrimethylammonium salts, benzyltriethylammonium salts, benzyltributylammonium salts, methyltrioctylammonium salts or tetrabutylammonium salts; and basic amino acid salts such as arginine salts, lysine salts, or histidine salts. Exemplary acid addition salts comprise, for example: mineral acid salts such as hydrochloride, hydrobromide, hydroiodide, sulfate salts (such as, e.g., sulfate or hydrogensulfate salts), nitrate salts, phosphate salts (such as, e.g., phosphate, hydrogenphosphate, or dihydrogenphosphate salts), carbonate salts, hydrogencarbonate salts, perchlorate salts, borate salts, or thiocyanate salts; organic acid salts such as acetate, propionate, butyrate, pentanoate, hexanoate, heptanoate, octanoate, cyclopentanepropionate, decanoate, undecanoate, oleate, stearate,

lactate, maleate, oxalate, fumarate, tartrate, malate, citrate, succinate, adipate, gluconate, glycolate, nicotinate, benzoate, salicylate, ascorbate, pamoate (embonate), camphorate, glucoheptanoate, or pivalate salts; sulfonate salts such as methanesulfonate (mesylate), ethanesulfonate (esylate), 2-hydroxyethanesulfonate (isethionate), benzenesulfonate (besylate), p-toluenesulfonate (tosylate), 2-naphthalenesulfonate (napsylate), 3-phenylsulfonate, or camphorsulfonate salts; glycerophosphate salts; and acidic amino acid salts such as aspartate or glutamate salts. Preferred pharmaceutically acceptable salts of the compound of formula (I) include a hydrochloride salt, a hydrobromide salt, a mesylate salt, a sulfate salt, a tartrate salt, a fumarate salt, an acetate salt, a citrate salt, and a phosphate salt. A particularly preferred pharmaceutically acceptable salt of the compound of formula (I) is a hydrochloride salt. Accordingly, it is preferred that the compound of formula (I), including any one of the specific compounds of formula (I) described herein, is in the form of a hydrochloride salt, a hydrobromide salt, a mesylate salt, a sulfate salt, a tartrate salt, a fumarate salt, an acetate salt, a citrate salt, or a phosphate salt, and it is particularly preferred that the compound of formula (I) is in the form of a hydrochloride salt. In one embodiment, the compound of formula (I) may be in the form of a formate salt.

The present invention also specifically relates to the compound of formula (I), including any one of the specific compounds of formula (I) described herein, in non-salt form.

Moreover, the scope of the invention embraces the compound of formula (I) in any solvated form, including, e.g., solvates with water (i.e., as a hydrate) or solvates with organic solvents such as, e.g., methanol, ethanol, isopropanol, acetic acid, ethyl acetate, ethanolamine, DMSO, or acetonitrile. All physical forms, including any amorphous or crystalline forms (i.e., polymorphs), of the compound of formula (I) are also encompassed within the scope of the invention. It is to be understood that such solvates and physical forms of pharmaceutically acceptable salts of the compound of the formula (I) are likewise embraced by the invention.

Furthermore, the compound of formula (I) may exist in the form of different isomers, in particular stereoisomers (including, e.g., geometric isomers (or cis/trans isomers), enantiomers and diastereomers) or tautomers (including, in particular, prototropic tautomers, such as keto/enol tautomers or thione/thiol tautomers). All such isomers of the compounds of formula (I) are contemplated as being part of the present invention, either in admixture or in pure or substantially pure form. As for stereoisomers, the invention embraces the isolated optical isomers of the compounds according to the invention as well as any mixtures thereof (including, in particular, racemic mixtures/racemates). The racemates can be resolved by physical methods, such as, e.g., fractional crystallization, separation or crystallization of diastereomeric derivatives, or separation by chiral column chromatography. The individual optical isomers can also be obtained from the racemates via salt formation with an optically active acid followed by crystallization. The present invention further encompasses any tautomers of the compound of formula (I). It will be understood that some compounds may exhibit tautomerism. In such cases, the formulae provided herein expressly depict only one of the possible tautomeric forms. The formulae and chemical names as provided herein are intended to encompass any tautomeric form of the corresponding compound and not to be limited merely to the specific tautomeric form depicted by the drawing or identified by the name of the compound.

The scope of the invention also embraces compounds of formula (I), in which one or more atoms are replaced by a specific isotope of the corresponding atom. For example, the invention encompasses compounds of formula (I), in which one or more hydrogen atoms (or, e.g., all hydrogen atoms) are replaced by deuterium atoms (i.e., $^{2}H$; also referred to as "D"). Accordingly, the invention also embraces compounds of formula (I) which are enriched in deuterium. Naturally occurring hydrogen is an isotopic mixture comprising about 99.98 mol-% hydrogen-1 ($^{1}H$) and about 0.0156 mol-% deuterium ($^{2}H$ or D). The content of deuterium in one or more hydrogen positions in the compounds of formula (I) can be increased using deuteration techniques known in the art. For example, a compound of formula (I) or a reactant or precursor to be used in the synthesis of the compound of formula (I) can be subjected to an H/D exchange reaction using, e.g., heavy water ($D_2O$). Further suitable deuteration techniques are described in: Atzrodt J et al., Bioorg Med Chem, 20(18), 5658-5667, 2012; William JS et al., Journal of Labelled Compounds and Radiopharmaceuticals, 53(11-12), 635-644, 2010; Modvig A et al., J Org Chem, 79, 5861-5868, 2014. The content of deuterium can be determined, e.g., using mass spectrometry or NMR spectroscopy. Unless specifically indicated otherwise, it is preferred that the compound of formula (I) is not enriched in deuterium. Accordingly, the presence of naturally occurring hydrogen atoms or $^{1}H$ hydrogen atoms in the compounds of formula (I) is preferred.

The present invention also embraces compounds of formula (I), in which one or more atoms are replaced by a positron-emitting isotope of the corresponding atom, such as, e.g., $^{18}F$, $^{11}C$, $^{13}N$, $^{15}O$, $^{76}Br$, $^{77}Br$, $^{120}I$ and/or $^{124}I$. Such compounds can be used as tracers, trackers or imaging probes in positron emission tomography (PET). The invention thus includes (i) compounds of formula (I), in which one or more fluorine atoms (or, e.g., all fluorine atoms) are replaced by $^{18}F$ atoms, (ii) compounds of formula (I), in which one or more carbon atoms (or, e.g., all carbon atoms) are replaced by $^{11}C$ atoms, (iii) compounds of formula (I), in which one or more nitrogen atoms (or, e.g., all nitrogen atoms) are replaced by $^{13}N$ atoms, (iv) compounds of formula (I), in which one or more oxygen atoms (or, e.g., all oxygen atoms) are replaced by $^{15}O$ atoms, (v) compounds of formula (I), in which one or more bromine atoms (or, e.g., all bromine atoms) are replaced by $^{76}Br$ atoms, (vi) compounds of formula (I), in which one or more bromine atoms (or, e.g., all bromine atoms) are replaced by $^{77}Br$ atoms, (vii) compounds of formula (I), in which one or more iodine atoms (or, e.g., all iodine atoms) are replaced by $^{120}I$

atoms, and (viii) compounds of formula (I), in which one or more iodine atoms (or, e.g., all iodine atoms) are replaced by $^{124}I$ atoms. In general, it is preferred that none of the atoms in the compounds of formula (I) are replaced by specific isotopes.

**Pharmaceutical compositions**

The compounds provided herein may be administered as compounds per se or may be formulated as medicaments. The medicaments/pharmaceutical compositions may optionally comprise one or more pharmaceutically acceptable excipients, such as carriers, diluents, fillers, disintegrants, lubricating agents, binders, colorants, pigments, stabilizers, preservatives, antioxidants, and/or solubility enhancers.

The pharmaceutical compositions may comprise one or more solubility enhancers, such as, e.g., poly(ethylene glycol), including poly(ethylene glycol) having a molecular weight in the range of about 200 to about 5,000 Da (e.g., PEG 200, PEG 300, PEG 400, or PEG 600), ethylene glycol, propylene glycol, glycerol, a non-ionic surfactant, tyloxapol, polysorbate 80, macrogol-15-hydroxystearate (e.g., Kolliphor® HS 15, CAS 70142-34-6), a phospholipid, lecithin, dimyristoyl phosphatidylcholine, dipalmitoyl phosphatidylcholine, distearoyl phosphatidylcholine, a cyclodextrin, α-cyclodextrin, β-cyclodextrin, γ-cyclodextrin, hydroxyethyl-β-cyclodextrin, hydroxypropyl-β-cyclodextrin, hydroxyethyl-γ-cyclodextrin, hydroxypropyl-γ-cyclodextrin, dihydroxypropyl-β-cyclodextrin, sulfobutylether-β-cyclodextrin, sulfobutylether-γ-cyclodextrin, glucosyl-α-cyclodextrin, glucosyl-β-cyclodextrin, diglucosyl-β-cyclodextrin, maltosyl-α-cyclodextrin, maltosyl-β-cyclodextrin, maltosyl-γ-cyclodextrin, maltotriosyl-β-cyclodextrin, maltotriosyl-γ-cyclodextrin, dimaltosyl-β-cyclodextrin, methyl-β-cyclodextrin, a carboxyalkyl thioether, hydroxypropyl methylcellulose, hydroxypropylcellulose, polyvinylpyrrolidone, a vinyl acetate copolymer, vinyl pyrrolidone, sodium lauryl sulfate, dioctyl sodium sulfosuccinate, or any combination thereof.

The pharmaceutical compositions may also comprise one or more preservatives, particularly one or more antimicrobial preservatives, such as, e.g., benzyl alcohol, chlorobutanol, 2-ethoxyethanol, m-cresol, chlorocresol (e.g., 2-chloro-3-methyl-phenol or 4-chloro-3-methyl-phenol), benzalkonium chloride, benzethonium chloride, benzoic acid (or a pharmaceutically acceptable salt thereof), sorbic acid (or a pharmaceutically acceptable salt thereof), chlorhexidine, thimerosal, or any combination thereof.

The pharmaceutical compositions can be formulated by techniques known to the person skilled in the art, such as the techniques published in "Remington: The Science and Practice of Pharmacy", Pharmaceutical Press, 22nd edition. The pharmaceutical compositions can be formulated as dosage forms for oral, parenteral, such as intramuscular, intravenous, subcutaneous, intradermal, intraarterial, intracardial, rectal, nasal, topical, aerosol or vaginal administration. Dosage forms for oral administration include coated and uncoated tablets, soft gelatin capsules, hard gelatin capsules, lozenges, troches, solutions, emulsions, suspensions, syrups, elixirs, powders and granules for reconstitution, dispersible powders and granules, medicated gums, chewing tablets and effervescent tablets. Dosage forms for parenteral administration include solutions, emulsions, suspensions, dispersions and powders and granules for reconstitution. Emulsions are a preferred dosage form for parenteral administration. Dosage forms for rectal and vaginal administration include suppositories and ovula. Dosage forms for nasal administration can be administered via inhalation and insufflation, for example by a metered inhaler. Dosage forms for topical administration include creams, gels, ointments, salves, patches and transdermal delivery systems.

The compounds of formula (I) or the above described pharmaceutical compositions comprising a compound of formula (I) may be administered to a subject by any convenient route of administration, whether systemically/peripherally or at the site of desired action, including but not limited to one or more of: oral (e.g., as a tablet, capsule, or as an ingestible solution), topical (e.g., transdermal, intranasal, ocular, buccal, and sublingual), parenteral (e.g., using injection techniques or infusion techniques, and including, for example, by injection, e.g., subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, or intrasternal by, e.g., implant of a depot, for example, subcutaneously or intramuscularly), pulmonary (e.g., by inhalation or insufflation therapy using, e.g., an aerosol, e.g., through mouth or nose), gastrointestinal, intrauterine, intraocular, subcutaneous, ophthalmic (including intravitreal or intracameral), rectal, or vaginal administration.

If said compounds or pharmaceutical compositions are administered parenterally, then examples of such administration include one or more of: intravenously, intraarterially, intraperitoneally, intrathecally, intraventricularly, intraurethrally, intrasternally, intracardially, intracranially, intramuscularly or subcutaneously administering the compounds or pharmaceutical compositions, and/or by using infusion techniques. For parenteral administration, the compounds are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well known to those skilled in the art.

Said compounds or pharmaceutical compositions can also be administered orally in the form of tablets, capsules, ovules, elixirs, solutions or suspensions, which may contain flavoring or coloring agents, for immediate-, delayed-,

modified-, sustained-, pulsed- or controlled-release applications.

The tablets may contain excipients such as microcrystalline cellulose, lactose, sodium citrate, calcium carbonate, dibasic calcium phosphate and glycine, disintegrants such as starch (preferably corn, potato or tapioca starch), sodium starch glycolate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia. Additionally, lubricating agents such as magnesium stearate, stearic acid, glyceryl behenate and talc may be included. Solid compositions of a similar type may also be employed as fillers in gelatin capsules. Preferred excipients in this regard include lactose, starch, a cellulose, or high molecular weight polyethylene glycols. For aqueous suspensions and/or elixirs, the agent may be combined with various sweetening or flavoring agents, coloring matter or dyes, with emulsifying and/or suspending agents and with diluents such as water, ethanol, propylene glycol and glycerin, and combinations thereof.

For oral administration, the compounds or pharmaceutical compositions are preferably administered by oral ingestion, particularly by swallowing. The compounds or pharmaceutical compositions can thus be administered to pass through the mouth into the gastrointestinal tract, which can also be referred to as "oral-gastrointestinal" administration.

Alternatively, said compounds or pharmaceutical compositions can be administered in the form of a suppository or pessary, or may be applied topically in the form of a gel, hydrogel, lotion, solution, cream, ointment or dusting powder. The compounds of the present invention may also be dermally or transdermally administered, for example, by the use of a skin patch.

Said compounds or pharmaceutical compositions may also be administered by sustained release systems. Suitable examples of sustained-release compositions include semi-permeable polymer matrices in the form of shaped articles, e.g., films, or microcapsules. Sustained-release matrices include, e.g., polylactides, copolymers of L-glutamic acid and gamma-ethyl-L-glutamate, poly(2-hydroxyethyl methacrylate), ethylene vinyl acetate, or poly-D-(-)-3-hydroxybutyric acid. Sustained-release pharmaceutical compositions also include liposomally entrapped compounds. The present invention thus also relates to liposomes containing a compound of the invention.

Said compounds or pharmaceutical compositions may also be administered by the pulmonary route, rectal routes, or the ocular route. For ophthalmic use, they can be formulated as micronized suspensions in isotonic, pH adjusted, sterile saline, or, preferably, as solutions in isotonic, pH adjusted, sterile saline, optionally in combination with a preservative such as a benzalkonium chloride. Alternatively, they may be formulated in an ointment such as petrolatum.

It is also envisaged to prepare dry powder formulations of the compounds of formula (I) for pulmonary administration, particularly inhalation. Such dry powders may be prepared by spray drying under conditions which result in a substantially amorphous glassy or a substantially crystalline bioactive powder. Accordingly, dry powders of the compounds of the present invention can be made according to an emulsification/spray drying process.

For topical application to the skin, said compounds or pharmaceutical compositions can be formulated as a suitable ointment containing the active compound suspended or dissolved in, for example, a mixture with one or more of the following: mineral oil, liquid petrolatum, white petrolatum, propylene glycol, emulsifying wax and water. Alternatively, they can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, 2-octyldodecanol, benzyl alcohol and water.

The present invention thus relates to the compounds or the pharmaceutical compositions provided herein, wherein the corresponding compound or pharmaceutical composition is to be administered by any one of: an oral route; topical route, including by transdermal, intranasal, ocular, buccal, or sublingual route; parenteral route using injection techniques or infusion techniques, including by subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, intrasternal, intraventricular, intraurethral, or intracranial route; pulmonary route, including by inhalation or insufflation therapy; gastrointestinal route; intrauterine route; intraocular route; subcutaneous route; ophthalmic route, including by intravitreal, or intracameral route; rectal route; or vaginal route. Preferred routes of administration are oral administration or parenteral administration. For each of the compounds or pharmaceutical compositions provided herein, it is particularly preferred that the respective compound or pharmaceutical composition is to be administered orally (particularly by oral ingestion).

Typically, a physician will determine the actual dosage which will be most suitable for an individual subject. The specific dose level and frequency of dosage for any particular individual subject may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the individual subject undergoing therapy.

A proposed, yet non-limiting dose of the compounds according to the invention for oral administration to a human (of approximately 70 kg body weight) may be 0.05 to 2000 mg, preferably 0.1 mg to 1500 mg, more preferably 0.1 mg to 1000 mg, of the active ingredient per unit dose. The unit dose may be administered, e.g., 1 to 3 times per day. The unit dose may also be administered 1 to 7 times per week, e.g., with not more than one administration per day. It will be appreciated that it may be necessary to make routine variations to the dosage depending on the age and weight of the patient/subject as

well as the severity of the condition to be treated. The precise dose and also the route of administration will ultimately be at the discretion of the attendant physician or veterinarian.

**Therapeutic use**

In one embodiment, the present invention relates to the compound of formula (I), or a pharmaceutically acceptable salt, hydrate or solvate thereof, or a pharmaceutical composition as defined herein for use in therapy.
As understood herein, there reference to the compound of formula (I), or a pharmaceutically acceptable salt, hydrate or solvent thereof is preferably a reference to the compound of formula (I) or a pharmaceutically acceptable salt thereof.
The present invention provides compounds that function as inhibitors of PARG. Thus, the present invention provides a method of inhibiting PARG enzyme activity in vitro or in vivo, said method comprising contacting a cell with an effective amount of the compound of formula (I), or a pharmaceutically acceptable salt, hydrate or solvate thereof, as defined herein.
The present invention also provides a method of selectively inhibiting PARG enzyme activity over PARP1 or ARH3 enzyme activity in vitro or in vivo. The said method comprises the steps of contacting a cell with an effective amount of a compound, or a pharmaceutically acceptable salt, hydrate or solvate thereof, as defined herein.
In a further embodiment, the present invention relates to the compound of formula (I), as disclosed herein, for use in a method of treating a disease or disorder in which PARG activity is implicated in a subject or patient in need of such treatment. Said method of treatment comprises administering to said subject/patient a therapeutically effective amount of a compound of formula (I), or a pharmaceutically acceptable salt, hydrate or solvate thereof, or a pharmaceutical composition as defined herein. In other words, in one embodiment the present invention relates to the compound of formula (I), as disclosed herein, for use in treating a disease or disorder in which PARG activity is implicated.
In a further embodiment, the present invention relates to a method of inhibiting cell proliferation, in vitro or in vivo, said method comprising contacting a cell with an effective amount of the compound of formula (I), or a pharmaceutically acceptable salt, hydrate or solvate thereof, as defined herein. Thus, the present invention relates to the compound of formula (I) or a pharmaceutically acceptable salt thereof for use in of inhibiting cell proliferation, in vitro or in vivo.
Thus, in a further embodiment, the present invention relates to a method of treating a proliferative disorder in a subject or patient in need of such treatment. The said method of treating a proliferative disorder in a subject or patient in need thereof comprises administering to said subject/patient a therapeutically effective amount of the compound of formula (I), or a pharmaceutically acceptable salt, hydrate or solvate thereof, or a pharmaceutical composition as defined herein. Preferably as disclosed herein, the proliferative disorder is cancer. Thus, the present invention relates to a method of treating cancer in a subject or patient in need thereof. The said method of treating cancer in a subject or patient in need thereof comprises administering to said subject/patient a therapeutically effective amount of the compound of formula (I), or a pharmaceutically acceptable salt, hydrate or solvate thereof, or a pharmaceutical composition as defined herein. In a particular embodiment, the cancer is human cancer.
In one embodiment, the present invention relates to the compound of formula (I) or a pharmaceutically acceptable salt, hydrate or solvate thereof, for use in treating a proliferative disorder. Preferably as disclosed herein, the proliferative disorder is cancer. Therefore, the present invention relates to the compound of formula (I) or a pharmaceutically acceptable salt, hydrate or solvate thereof for use in treating cancer. In a particular embodiment, the cancer is human cancer.
In a further embodiment, the present invention relates to the compound of formula (I), or a pharmaceutically acceptable salt, hydrate or solvate thereof, as defined herein, for use in the manufacture of a medicament for the treatment of a proliferative condition. In a preferred embodiment, the proliferative condition is cancer, more preferably a human cancer. Thus, preferably the present invention relates to the compound of formula (I), or a pharmaceutically acceptable salt, hydrate or solvate thereof, as defined herein, for use in the manufacture of a medicament for the treatment of cancer, preferably for the treatment of human cancer.
In a further embodiment, the present invention relates to the compound of formula (I), or a pharmaceutically acceptable salt, hydrate or solvate thereof, as defined herein, for use in the manufacture of a medicament for the inhibition of PARG enzyme activity. Preferably, the inhibition of PARG enzyme activity is selective inhibition of PARG enzyme activity over PARP1 or ARH3 enzyme activity. Thus, the present invention relates to the compound of formula (I), or a pharmaceutically acceptable salt, hydrate or solvate thereof, as defined herein, for use in the manufacture of a medicament for the selective inhibition of PARG enzyme activity over PARP1 or ARH3 enzyme activity.
The present invention further provides the compound of formula (I), or a pharmaceutically acceptable salt, hydrate or solvate thereof, as defined herein for use in the manufacture of a medicament for the treatment of a disease or disorder in which PARG activity is implicated, as defined herein.
As understood herein, the term "proliferative disorder" are used interchangeably herein and pertain to an unwanted or uncontrolled cellular proliferation of excessive or abnormal cells which is undesired, such as, neoplastic or

hyperplastic growth, whether in vitro or in vivo. Examples of proliferative conditions include, but are not limited to, pre-malignant and malignant cellular proliferation, including but not limited to, malignant neoplasms and tumours, cancers, leukemias, psoriasis, bone diseases, fibroproliferative disorders (e.g., of connective tissues), and atherosclerosis. Any type of cell may be treated, including but not limited to, lung, colon, breast, ovarian, prostate, liver, pancreas, brain, and skin.

The anti-proliferative effects of the compound of formula (I) of the present invention have particular application in the treatment of human cancers (by virtue of their inhibition of PARG enzyme activity). The anti-cancer effect may arise through one or more mechanisms, including but not limited to, the regulation of cell proliferation, the inhibition of angiogenesis (the formation of new blood vessels), the inhibition of metastasis (the spread of a tumour from its origin), the inhibition of invasion (the spread of tumour cells into neighbouring normal structures), or the promotion of apoptosis (programmed cell death).

The antiproliferative treatment with the compound of formula (I) or a pharmaceutically acceptable salt, hydrate or solvate thereof, as defined hereinbefore, may be applied as a sole therapy or may involve, in addition to the compound of the invention, conventional surgery or radiotherapy or chemotherapy. Such chemotherapy may include one or more of the following categories of anti-tumour agents:-

(i) other anti proliferative/antineoplastic drugs and combinations thereof, as used in medical oncology, such as alkylating agents (for example cis-platin, oxaliplatin, carboplatin, cyclophosphamide, nitrogen mustard, melphalan, chlorambucil, busulphan, temozolomide and nitrosoureas); antimetabolites (for example gemcitabine and antifolates such as fluoropyrimidines like 5-fluorouracil and tegafur, raltitrexed, methotrexate, cytosine arabinoside, and hydroxyurea); antitumour antibiotics (for example anthracyclines like adriamycin, bleomycin, doxorubicin, daunomycin, epirubicin, idarubicin, mitomycin-C, dactinomycin and mithramycin); antimitotic agents (for example vinca alkaloids like vincristine, vinblastine, vindesine and vinorelbine and taxoids like taxol and taxotere and polokinase inhibitors); and topoisomerase inhibitors (for example epipodophyllotoxins like etoposide and teniposide, amsacrine, topotecan and camptothecin);

(ii) cytostatic agents such as antioestrogens (for example tamoxifen, fulvestrant, toremifene, raloxifene, droloxifene and iodoxyfene), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrozole, vorazole and exemestane) and inhibitors of 5oc-reductase such as finasteride;

(iii) anti-invasion agents [for example c-Src kinase family inhibitors like 4-(6-chloro-2,3-methylenedioxyanilino)-7-[2-(4-methylpiperazin-1 -yl)ethoxy]-5-tetrahydropyran-4- yloxyquinazoline (AZD0530; International Patent Application WO 01/94341 ), N-(2-chloro-6- methylphenyl)-2-{6-[4-(2-hydroxyethyl)piperazin-1 -yl]-2-methylpyrimidin-4-ylamino}thiazole- 5-carboxamide (dasatinib, BMS-354825; J. Med. Chem., 2004, 47, 6658-6661 ) and bosutinib (SKI-606), and metalloproteinase inhibitors like marimastat, inhibitors of urokinase plasminogen activator receptor function or antibodies to Heparanase];

(iv) inhibitors of growth factor function: for example such inhibitors include growth factor antibodies and growth factor receptor antibodies (for example the anti-erbB2 antibody trastuzumab [Herceptin™], the anti-EGFR antibody panitumumab, the anti-erbB1 antibody cetuximab [Erbitux, C225] and any growth factor or growth factor receptor antibodies disclosed by Stern et al. (Critical reviews in oncology/haematology, 2005, Vol. 54, pp1 1 -29); such inhibitors also include tyrosine kinase inhibitors, for example inhibitors of the epidermal growth factor family (for example EGFR family tyrosine kinase inhibitors such as N-(3-chloro- 4-fluorophenyl)-7-methoxy-6-(3-morpholinopropoxy)quinazolin-4-amine (gefitinib, ZD1839), N-(3-ethynylphenyl)-6,7-bis(2-methoxyethoxy)quinazolin-4-amine (erlotinib, OSI-774) and 6- acrylamido-N-(3-chloro-4-fluorophenyl)-7-(3-morpholinopropoxy)-quinazolin-4-amine (CI 1033), erbB2 tyrosine kinase inhibitors such as lapatinib); inhibitors of the hepatocyte growth factor family; inhibitors of the insulin growth factor family; inhibitors of the platelet-derived growth factor family such as imatinib and/or nilotinib (AMN107); inhibitors of serine/threonine kinases (for example Ras/Raf signalling inhibitors such as farnesyl transferase inhibitors, for example sorafenib (BAY 43-9006), tipifarnib (R1 15777) and lonafarnib (SCH66336)), inhibitors of cell signalling through MEK and/or AKT kinases, c-kit inhibitors, abl kinase inhibitors, PI3 kinase inhibitors, PIt3 kinase inhibitors, CSF-1 R kinase inhibitors, IGF receptor (insulin-like growth factor) kinase inhibitors; aurora kinase inhibitors (for example AZD1 152, PH739358, VX-680, MLN8054, R763, MP235, MP529, VX-528 AND AX39459) and cyclin dependent kinase inhibitors such as CDK2 and/or CDK4 inhibitors;

(v) antiangiogenic agents such as those which inhibit the effects of vascular endothelial growth factor, [for example the anti-vascular endothelial cell growth factor antibody bevacizumab (Avastin™) and for example, a VEGF receptor tyrosine kinase inhibitor such as vandetanib (ZD6474), vatalanib (PTK787), sunitinib (SU1 1248), axitinib (AG-013736), pazopanib (GW 786034) and 4-(4-fluoro-2-methylindol-5-yloxy)-6-methoxy-7-(3-pyrrolidin-1 - ylpropoxy)quinazoline (AZD2171 ; Example 240 within WO 00/47212), compounds such as those disclosed in International Patent Applications WO97/22596, WO 97/30035, WO 97/32856 and WO 98/13354 and compounds that work

by other mechanisms (for example linomide, inhibitors of integrin $\alpha v\beta 3$ function and angiostatin)];
(vi) vascular damaging agents such as Combretastatin A4 and compounds disclosed in International Patent Applications WO 99/02166, WO 00/40529, WO 00/41669, WO 01 /92224, WO 02/04434 and WO 02/08213; (vii) an endothelin receptor antagonist, for example zibotentan (ZD4054) or atrasentan;
(viii) antisense therapies, for example those which are directed to the targets listed above, such as ISIS 2503, an anti-ras antisense;
(ix) gene therapy approaches, including for example approaches to replace aberrant genes such as aberrant p53 or aberrant BRCA1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multidrug resistance gene therapy; and
(x) immunotherapy approaches, including for example ex-vivo and in-vivo approaches to increase the immunogenicity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines and approaches using anti-idiotypic antibodies.

In a particular embodiment, the antiproliferative treatment defined hereinbefore may involve, in addition to the compound of formula (I) of the invention, conventional surgery or radiotherapy or chemotherapy. Such conjoint treatment may be achieved by way of the simultaneous, sequential or separate dosing of the individual components of the treatment. Such combination products employ the compounds of this invention within the dosage range described hereinbefore and the other pharmaceutically-active agent within its approved dosage range.
According to this aspect the present invention further relates to the compound of formula (I) or a pharmaceutically acceptable salt, hydrate or solvate thereof, as defined herein, for use in the treatment of a cancer (for example a cancer involving a solid tumour) in combination with another anti-tumour agent. The anti-tumour agent is preferably selected from the anti-tumour agents as listed hereinabove.
As understood herein, the term "combination" refers to simultaneous, separate or sequential administration. In one aspect of the invention "combination" refers to simultaneous administration. In another aspect of the invention "combination" refers to separate administration. In a further aspect of the invention "combination" refers to sequential administration. Where the administration is sequential or separate, the delay in administering the second component should not be such as to lose the beneficial effect of the combination.

## Examples

The following examples are merely illustrative of the present invention and should not be construed to limit the scope of the invention which is defined by the appended claims.

## Synthesis of the compound of formula (I)

The syntheses of the compounds of formula (I) according to the present invention are preferably carried out as described in, or per analogy to, specific synthetic procedures described in the following synthetic examples.

## Preparative examples

### General considerations

Abbreviations used in the descriptions that follow are: AcOH (acetic acid); aq. (aqueous); Ar (Argon); Atm (atmosphere); $BH_3$.THF (boran tetrahydrofuran complex); br. (broad, $^1$H NMR signal); $Boc_2O$ (di-tert-butyldicarbonate); (Cataxium APdG$_3$ (Mesylate[(di(1-adamantyl)-n-butylphosphine)-2-(2'-amino-1,1'-biphenyl)]palladium(II)); ($CDCl_3$ (deuterated chloroform); cHex (cyclohexane); CMPB ( Cyanomethylene trimethylphosphorane); $Cs_2CO_3$ (cesium carbonate); CuI (copper iodide); DABCO ((1,4-diazabicyclo[2.2.2]octane)); DAST (diethylaminosulfur trifluoride);DBU (1,8-Diazabicyclo(5.4.0)undec-7-ene); DCE (dichloroethane); d (doublet, $^1$H NMR signal); DCM (dichloromethane); DIBAL-H (diisobutyl aluminium hydride); DIPEA or DIEA (di-*iso*-propylethylamine); DMAP (4- *N*-*N*-dimethylaminopyridine), DME (1,2-dimethoxyethane), DMEDA (dimethylethylenediamine ); DMF (*N*-*N*-dimethylformamide); DMSO (dimethyl sulfoxide); DPPA (diphenylphosphoride azide);dtbbpy (Bis(1,1-dimethylethyl)-2,2'-bipyridine); ES (electrospray); EtOAc or EA (ethyl acetate); EtOH (ethanol); h (hour(s)); FA (formic acid); HATU (1-[Bis(dimethylamino)methylene]-1H-1,2,3-triazolo [4,5-b]pyridinium 3-oxide hexafluorophosphate); HFIP ( Hexafluoroisopropanol); $^1$H NMR (proton nuclear magnetic resonance spectroscopy); HPLC (High Performance Liquid Chromatography), iPrOH (*iso*-propanol); $K_3PO_4$ (tripotassium phosphate); Ir[dF($CF_3$)(dtbbpy)$PF_6$ ((4,4'-Di-t-butyl-2,2'-bipyridine)bis[3,5-difluoro-2-[5-trifluoromethyl-2-pyridinyl-kN)phenyl-kC]iridium(III) hexafluorophosphate); LiOH (lithium hydroxide); m (multiplet, $^1$H NMR signal); mCPBA (*meta*-chloroperoxybenzoic acid), MeCN (acetonitrile), MeOH (methanol); min (minute(s)); $MnO_2$ (Manganese (IV) oxide); MS (mass spectrometry); MTBE (methyl *tert*-butyl ether); $NaBH_4$ (sodium borohydride); $NaHCO_3$ (sodium hydrogenocarbonate); $Na_2S_2O_3$ (sodium thiosulfate); NCS (N-chlorosuccinimide); $NH_3$ (ammonia); $NH_4Cl$ (ammonium fluoride); $NiCl_2$ (nickel dichloride); NIS (N-lodosuccinimide); NMP (N-methylpyrrolidone); NMR (nuclear magnetic resonance); Pd/C (palladium on charcoal); $Pd_2dba_3$ (tris(dibenzylideneacetone)dipalladium ); Pd(dppf)$Cl_2$ (1,1 - Bis(diphenylphosphino) ferrocene dichloropalladium ); $Pd(Ph_3)_2Cl_2$ (Bis(triphenylphosphine)palladium(II) dichloride ); PE (petroleum ether); Pd-PEPPSI-IPentCl o-picoline ([1,3-bis[2,6-bis(1-ethylpropyl)phenyl]-4,5-dichloro-imidazol-2-ylidene]-dichloro-(2-methylpyridin-1-ium-1-yl)palladium; $Pd(OH)_2$ (palladium hydroxide); $Pd(Ph_3)_4$ (Palladium-tetrakis(triphenylphosphine)); $PhI(OAc)_2$ ((Diacetoxyiodo)benzene)); $P(tBu)_3$ (Tri-tert-butylphosphine ); Py (pyridine); q (quartet, 1H NMR signal); quin (quintet, 1H NMR signal); rac (racemic); RT (retention time); s (singlet, $^1$H NMR signal); sat. (saturated); t (triplet, $^1$H NMR signal); TBAF (tetrabutylammonium fluoride); *tert*-BuBrettPhos-Pd-G3 ([(2-Di-tert-butylphosphino-3,6-dimethoxy-2',4',6'-triisopropyl-1,1'-biphenyl)-2-(2'-amino-1,1'-biphenyl)]palladium(II) methanesulfonate); tBuXPhos Pd G3 (Methanesulfonato(2-di-t-butylphosphino-2',4',6'-tri-i-propyl-1,1'-biphenyl)(2'-amino-1,1'-biphenyl-2-yl)palladium(II))TBDMSCl or TBSCl (tert-butyldimethylsilyl chloride); tBuOH (tert-butanol); TEA (triethylamine) ; TFA (trifluoroacetic acid); TFAA (trifluoroacetic anhydride), THF (tetrahydrofuran); TLC (thin layer chromatography); $TMSCHN_2$ (Trimethylsilyldiazomethane); TMSCN (trimethylsilyl cyanide); TMSOTf (Trimethylsilyl trifluoromethanesulfonate ); TTMSS (trimethylsilane); UPLC (Ultra-High Performance Liquid Chromatography), UV (ultraviolet), wt-% (percent by weight); Xantphos (4,5-Bis(diphenylphosphino)-9,9-dimethylxanthene); Xantphos Pd G4 (Methanesulfonato[9,9-dimethyl-4,5-bis(diphenylphosphino)xanthene](2'-methylamino-1,1'-biphenyl-2-yl)palladium(II)).

General Procedure: All starting materials and solvents were obtained either from commercial sources or prepared according to literature references. Commercially available reagents and anhydrous solvents were used as supplied, without further purification. Unless otherwise stated all reactions were stirred. Organic solutions were routinely dried over anhydrous sodium sulfate. Column chromatography was performed on pre-packed silica (100-1000 mesh, 40-63 μm) cartridges using the amount indicated. All air- and moisture-sensitive reactions were carried out in oven-dried (at 120 °C) glassware under an inert atmosphere of nitrogen or argon. Compound names were generated using ChemDraw Prime (Perkin Elmer). In some cases generally accepted names of commercially available reagents were used in place of ChemDraw generated names.

Reversed Phase HPLC conditions for LCMS Analysis of final compounds:

Method 1: SHIMADZU LCMS-2020 Kinetex EVO C18 2.1X30mm,5μm at 50°C; Mobile Phase: A: 0.0375% TFA in water (v/v); B: 0.01875% TFA in MeCN (v/v); flow rate held at 1.5 mL/min; eluted with the mobile phase over 1.55 min employing UV detection at 220 nm and 254 nm. Gradient information: 0-0.80 min, ramped from 95% A-5% B to 5% A-95% B; 0.80-1.20 min, held at 5% A-95% B; 1.20-1.21 min, returned to 95% A-5% B, 1.21-1.55 min, held at 95% A-5% B.

Method 2: SHIMADZU LCMS-2020 Kinetex EVO C18 2.1X30mm,5μm at 40°C ; Mobile Phase : A: 0.025% $NH_3 \cdot H_2O$ in water (v/v) , B: MeCN; flow rate held at 1.5 mL/min; eluted with the mobile phase over 1.55 min employing UV detection at 220 nm and 254 nm. Gradient information: 0-0.80 min, ramped from 95% A-5% B to 5% A-95% B; 0.80-1.20 min, held at 5% A-95% B; 1.20-1.21 min, returned to 95% A-5% B, 1.21-1.55 min, held at 95% A-5% B.

Method 3: SHIMADZU LCMS-2020 Kinetex EVO C18 2.1X30mm,5μm at 50°C; Mobile Phase: A: 0.0375% TFA in water (v/v); B: 0.01875% TFA in MeCN (v/v); flow rate held at 2.0 mL/min; eluted with the mobile phase over 0.80 min employing UV detection at 220 nm and 254 nm. Gradient information: 0-0.80 min, ramped from 95% A-5% B to 5% A-95% B; 0.80-1.20 min, held at 5% A-95% B; 1.20-1.21 min, returned to 95% A-5% B, 1.21-1.55 min, held at 95% A-5% B.

Method 4: SHIMADZU LCMS-2020 Kinetex® EVO C18 2.1X20 mm 2.6 um at 50°C; Mobile Phase: A: 0.0375% TFA in water (v/v); B: 0.01875% TFA in MeCN (v/v); flow rate held at 2.0 mL/min; eluted with the mobile phase over 1.00 min employing UV detection at 220 nm and 254 nm. Gradient information: 0.01-0.60 min, ramped from 95% A-5% B to 5% A-95% B; 0.61-0.78 min, held at 5%A-95% B; 0.78-0.79 min, returned to 95% A-5% B, 0.79-0.80 min, held at 95% A-5% B.

$^1$H NMR Spectroscopy:

$^1$H NMR spectra were acquired on a Bruker Avance III spectrometer at 400 MHz using residual undeuterated solvent as reference. $^1$H NMR signals are specified with their multiplicity / combined multiplicities as apparent from the spectrum; possible higher-order effects are not considered. Chemical shifts of the signals (δ) are specified as ppm (parts per million).

Salt stoichiometry:

In the present text, in particular in the experimental section, for the synthesis of intermediates and of examples of

the present invention, when a compound is mentioned as a salt form with the corresponding base or acid, the exact stoichiometric composition of said salt form, as obtained by the respective preparation and/or purification process, is, in most cases, unknown. Unless specified otherwise, suffixes to chemical names or structural formulae such as "hydrochloride", "trifluoroacetate", "sodium salt", or "x HO", "x CF3COOH", "x Na+", for example, are to be understood as not a stoichiometric specification, but solely as a salt form. This applies analogously to cases in which synthesis intermediates or example compounds or salts thereof have been obtained, by the preparation and/or purification processes described, as solvates, such as hydrates with (if defined) unknown stoichiometric composition.

Preparation of Intermediate 1.1

(5-bromo-3-chloropyridin-2-yl)methanamine

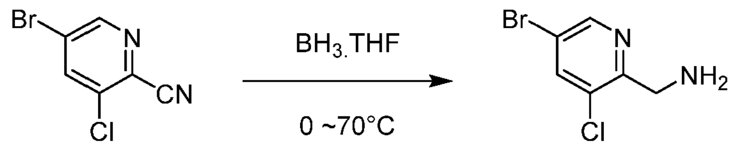


To a mixture of 5-bromo-3-chloropicolinonitrile (2.0 g, 9.20 mmol) in THF (10 mL) under ice-water cooling was added $BH_3 \cdot THF$ (1 M, 11.04 mL) over 5 min. The mixture was stirred at 0°C for 30 min before it was warmed to 20°C and stirred for another 30 min at this temperature. The mixture was cooled to 0°C and quenched with dropwise addition of MeOH (10 mL) over 5 min. The mixture was heated to 70°C and stirred for 30 min at this temperature. The reaction was concentrated under vacuum to give the crude product (2.2 g) as a light brown solid. The crude product was dissolved in HCl (aq. 2M, 20 mL), washed with DCM (20 mL; 2x) and the aqueous phase was finally concentrated under vacuum to give the product (5-bromo-3-chloro-2-pyridyl)methanamine (1.5 g, 4.07 mmol, 44.26% yield, 70% purity, HCl salt) as a light brown solid.

**RT** 0.18 min (**method 2**); **m/z** 222.9 $(M+H)^+$ $(ESI^+)$, $^1H$ NMR (400 MHz, DMSO-*d*6) $\delta$ = 8.78 (d, *J* = 2.0 Hz, 1H), 8.69 (br, 3H), 8.47 (d, *J* = 2.0 Hz, 1H), 4.24 (d, *J* = 6.2 Hz, 2H).

Preparation of Intermediate 1.2

Ethyl 2-(((5-bromo-3-chloropyridin-2-yl)methyl)amino)-2-oxoacetate

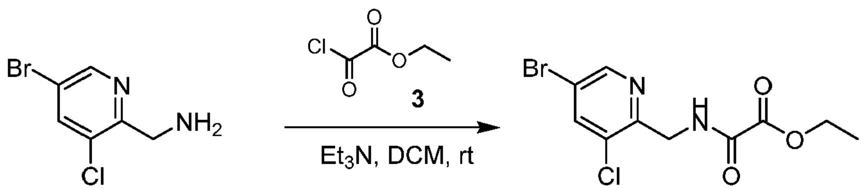


To a mixture of (5-bromo-3-chloro-2-pyridyl)methanamine (1.5 g, 5.82 mmol, HCl salt) in DCM (30 mL) under ice-water cooling was added DIPEA (2.25 g, 17.45 mmol). Then, ethyl 2-chloro-2-oxoacetate (952.77 mg, 6.98 mmol) was added over 5 min and the mixture was stirred at 0°C for 30 min. The mixture was warmed to 20°C and stirred for 30 min at this temperature. The mixture was quenched with aqueous $NaHCO_3$ solution (50 mL) and extracted with DCM (50 mL). The organic phase was separated, dried over $Na_2SO_4$, filtered and concentrated under vacuum. The residue was purified by column chromatography on silica gel (PE: EtOAc=10:1 to 1:1) to give the product ethyl 2-(((5-bromo-3-chloropyridin-2-yl)methyl)amino)-2-oxoacetate (1300 mg, 3.64 mmol, 62.57% yield, 65.6% purity) as a white solid. **RT** 0.61min (**method 1**); **m/z** 322.8 $(M+H)^+$ $(ESI^+)$. The product was used without further purification in the next step.

Preparation of Intermediate 1.3

Ethyl 6-bromo-8-chloroimidazo[1,5-a]pyridine-3-carboxylate

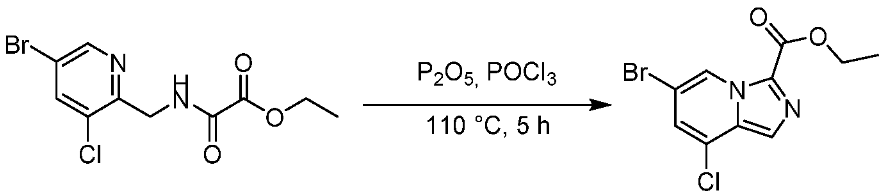


To a mixture of ethyl ethyl 2-(((5-bromo-3-chloropyridin-2-yl)methyl)amino)-2-oxoacetate (1300 mg, 4.04 mmol) in $POCl_3$ (15 mL) under ice water cooling was added phosphorus pentoxide (2.87 g, 20.21 mmol). The mixture was heated to 110°C and stirred for 5 h at this temperature. The mixture was cooled to 25°C and concentrated under vacuum to give a residue. The residue was dissolved in EtOAc (50 mL) and washed with water (30 mL) and an aqueous $NaHCO_3$ solution (30 mL). Then it was was finally concentrated under vacuum to give a residue. The residue was purified by column chromatography on silica gel (PE: EtOAc=10:1 to 3:1) to give the product ethyl 6-bromo-8-chloroimidazo[1,5-a]pyridine-3-carboxylate (900 mg, 2.97 mmol, 73.34% yield) as a white solid.

**RT** 0.718 min (**method 1**), **m/z** 304.8(M+H)$^+$ (ESI$^+$), $^1$H NMR (400 MHz, CHLOROFORM-*d*) $\delta$ = 9.47 (s, 1H), 7.77 (s, 1H), 7.20 (s, 1H), 4.65-4.42 (m, 2H), 1.57-1.42 (m, 3H)

Preparation of Intermediate 1.4

6-bromo-8-chloroimidazo[1,5-a]pyridine-3-carbohydrazide

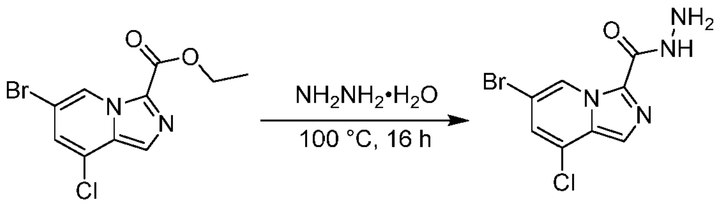


To a mixture of ethyl 6-bromo-8-chloroimidazo[1,5-a]pyridine-3-carboxylate (900 mg, 2.97 mmol) in EtOH (20 mL) was added $NH_2NH_2 \cdot H_2O$ (1.48 g, 29.65 mmol, 98%). The mixture was heated to 80°C and stirred for 2 h at this temperature. The reaction was cooled to 25°C and the precipitated solid was separated off. The crude product was triturated with EtOH (5 mL) to give 6-bromo-8-chloroimidazo[1,5-a]pyridine-3-carbohydrazide (650 mg, 2.25 mmol, 75.72% yield) as a white solid.

**RT** 0.56 min **(method** 1); **m/z** 290.8 (M+H)$^+$ (ESI$^+$); **$^1$H NMR** (400 MHz, DMSO-$d_6$) $\delta$ = 10.02 (s, 1H), 9.50 (s, 1H), 7.72 (s, 1H), 7.51 (s, 1H), 4.58 (d, *J* = 4.0 Hz, 2H).

Preparation of Intermediate 1.5

6-bromo-8-chloro-N'-(2,2-difluoroacetyl)imidazo[1,5-a]pyridine-3-carbohydrazide

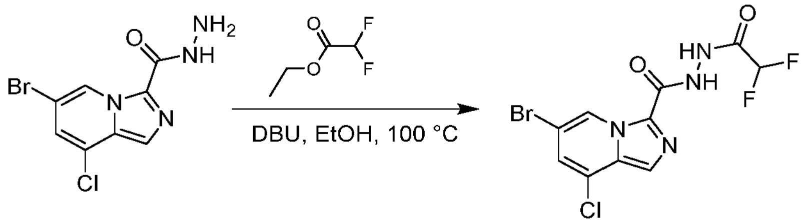


To a mixture of 6-bromo-8-chloroimidazo[1,5-a]pyridine-3-carbohydrazide (650 mg, 2.25 mmol) in EtOH (20 mL) was added ethyl 2,2-difluoroacetate (3.10 g, 22.45 mmol) and DBU (683.58 mg, 4.49 mmol). The mixture was heated to 100°C stirred for 16 h at this temperature. The mixture was cooled to 25°C and concentrated under vacuum. The residue was dissolved with DCM (50 mL), washed with an aqueous $NH_4Cl$ solution (30 mL; 2x) and concentrated under vacuum to give the crude product. The crude product was purified by column chromatography on silica gel (PE/EtOAc=1: 1 to MeOH:

EtOAc=1: 10) to give the product 6-bromo-8-chloro-N'-(2,2-difluoroacetyl)imidazo[1,5-a]pyridine-3-carbohydrazide (650 mg, 1.56 mmol, 69.32% yield, 88% purity) as a white solid.

**RT** 0.62 min (**method 1**); **m/z** 368.8 $(M+H)^+$ $(ESI^+)$; **$^1$H NMR** (400 MHz, DMSO-$d_6$) $\delta$ = 10.95 (br, 2H), 9.44 (s, 1H), 7.81 (s, 1H), 7.59 (s, 1H), 6.38 (t, *J* = 53.2, 1H).

Preparation of Intermediate 1.6

2-(6-bromo-8-chloroimidazo[1,5-a]pyridin-3-yl)-5-(difluoromethyl)-1,3,4-thiadiazole

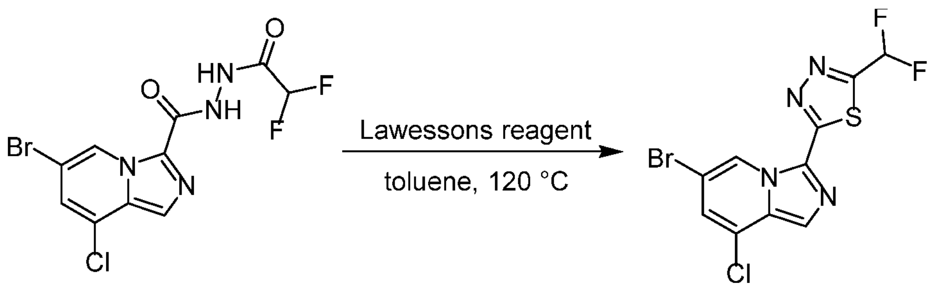


To a mixture of 6-bromo-8-chloro-N'-(2,2-difluoroacetyl)imidazo[1,5-a]pyridine-3-carbohydrazide (550 mg, 1.50 mmol) in toluene (20 mL) was added Lawessons reagent (665.80 mg, 1.65 mmol) under a $N_2$ atmosphere. The reaction was heated to 120°C and stirred for 2 h at this temperature. The reaction was cooled to 25°C and concentrated under vacuum. The residue was triturated with MeOH (10 mL) at 70°C for 1h, filtered and the cake was collected, and dried under vacuum to give the product 2-(6-bromo-8-chloroimidazo[1,5-a]pyridin-3-yl)-5-(difluoromethyl)-1,3,4-thiadiazole (530 mg, 1.45 mmol, 96.88% yield) as a light yellow solid.

**RT** 0.806 min (**method 1**); **m/z** 366.8 $(M+H)^+$ $(ESI^+)$; **$^1$H NMR** (400 MHz, DMSO-$d_6$) $\delta$ = 9.62 (s, 1H), 8.64 (s, 1H), 8.09 (s, 1H), 7.70 (t, *J* = 53.2, 1H).

Preparation of Intermediate 1.7

2-(6-(benzylthio)-8-chloroimidazo[1,5-a]pyridin-3-yl)-5-(difluoromethyl)-1,3,4-thiadiazole

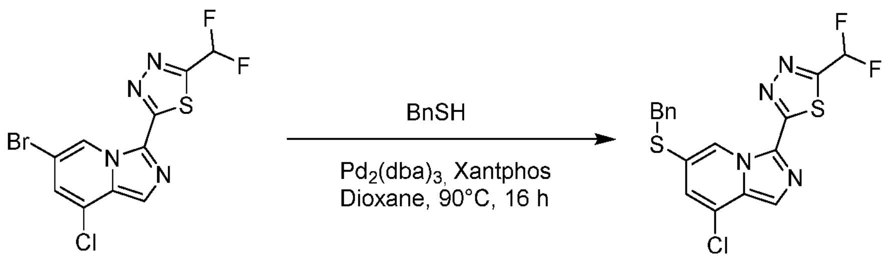


To a mixture of 2-(6-bromo-8-chloroimidazo[1,5-a]pyridin-3-yl)-5-(difluoromethyl)-1,3,4-thiadiazole (450 mg, 1.23 mmol) and phenylmethanethiol (168.17 mg, 1.35 mmol) in dioxane (10 mL) which was degassed with nitrogen for 2 min was added $Pd_2(dba)_3$ (112.72 mg, 123.09 μmol), Xantphos (71.22 mg, 123.09 μmol) and DIEA (477.26 mg, 3.69 mmol) under nitrogen. The mixture was heated to 90°C and stirred for 16 h at this temperature. The mixture was filtered and concentrated under vacuum. The residue was purified by column chromatography on silica gel (PE: EtOAc=20:1 to 5:1) to give the product 2-(6-(benzylthio)-8-chloroimidazo[1,5-a]pyridin-3-yl)-5-(difluoromethyl)-1,3,4-thiadiazole (250 mg, 489.15 μmol, 39.74% yield, 80% purity) as a light yellow solid.

**RT** 0.99 min (**method 1**); **m/z** 409.0 $(M+H)^+$ $(ESI^+)$; **$^1$H NMR** (400 MHz, CHLOROFORM-d) $\delta$ = 9.35 (s, 1H), 7.69 - 7.67 (m, 1H), 7.39 - 7.28 (m, 2H), 7.25 - 7.12 (m, 3H), 7.05 (t, *J* = 53.2, 1H), 7.00 (s, 1H), 6.90 (s, 1H), 4.10 (s, 2H)

Preparation of Intermediate 1.8

2-(6-(benzylthio)-8-chloro-1-iodoimidazo[1,5-a]pyridin-3-yl)-5-(difluoromethyl)-1,3,4-thiadiazole

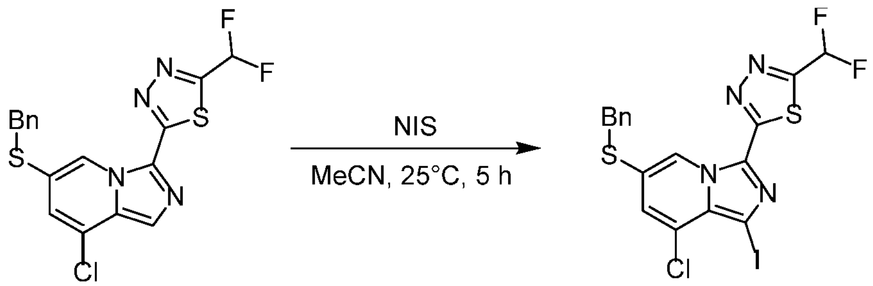


To a mixture of 2-(6-(benzylthio)-8-chloroimidazo[1,5-a]pyridin-3-yl)-5-(difluoromethyl)-1,3,4-thiadiazole (130 mg, 317.95 μmol) in MeCN (5 mL) at 0°C was added NIS (78.68 mg, 349.74 μmol). The mixture was stirred at 25°C for 5 h. The reaction mixture was used for the next step directly.

**RT** 0.99 min (**method 1**); **m/z** 535.0 $(M+H)^+$ $(ESI^+)$

Preparation of Intermediate 1.9

8-chloro-3-(5-(difluoromethyl)-1,3,4-thiadiazol-2-yl)-1-iodoimidazo[1,5-a]pyridine-6-sulfonyl chloride

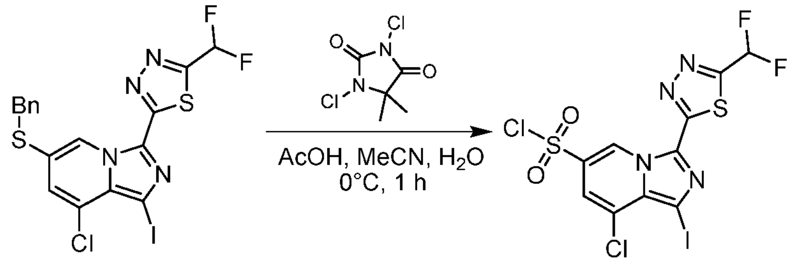


A mixture of 2-(6-(benzylthio)-8-chloro-1-iodoimidazo[1,5-a]pyridin-3-yl)-5-(difluoromethyl)-1,3,4-thiadiazole (170 mg, 317.95 μmol) in MeCN (5 mL) was cooled to 0°C before $H_2O$ (5.73 mg, 317.95 μmol), AcOH (38.19 mg, 635.89 μmol) and 1,3-dichloro-5,5-dimethylimidazolidine-2,4-dione (125.28 mg, 635.89 μmol) was added. The mixture was stirred at 0°C for 2 h. The mixture was diluted with THF (8 mL), dried over $Na_2SO_4$, filtered and concentrated under vacuum to give the crude product 8-chloro-3-(5-(difluoromethyl)-1,3,4-thiadiazol-2-yl)-1-iodoimidazo[1,5-a]pyridine-6-sulfonyl chloride (160 mg, 219.14 μmol, 68.92% yield, 70% purity) as a light brown oil.

It is noted that it cannot be excluded that the dichloro-compound 1,8-dichloro-3-(5-(difluoromethyl)-1,3,4-thiadiazol-2-yl)imidazo[1,5-a]pyridine-6-sulfonyl chloride was also formed in this process.

Preparation of Intermediate 1.10

8-chloro-3-(5-(difluoromethyl)-1,3,4-thiadiazol-2-yl)-1-iodo-N-(1-methylcyclopropyl)imidazo[1,5-a]pyridine-6-sulfonamide

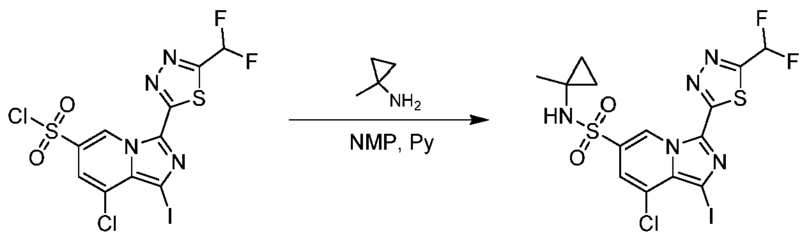

To a mixture of 1-methylcyclopropan-1-amine (37.80 mg, 531.49 μmol) in pyridine (1 mL) and NMP (N-methyl-2-pyrrolidon) (1 mL) at 0°C was added 8-chloro-3-(5-(difluoromethyl)-1,3,4-thiadiazol-2-yl)-1-iodoimidazo[1,5-a]pyridine-6-sulfonyl chloride (90 mg, 176.09 μmol) in MeCN (2 mL). The reaction was stirred at 0°C for 50 min. The reaction mixture was quenched with water (10 mL) and extracted with EtOAc (10 mL; 2x). The organic phase was collected, dried over $Na_2SO_4$, filtered and concentrated under vacuum to give a residue which was purified by preparative TLC (PE:EtOAc = 3:1) to give the product 8-chloro-3-(5-(difluoromethyl)-1,3,4-thiadiazol-2-yl)-1-iodo-N-(1-methylcyclopropyl)imidazo[1,5-a]pyridine-6-sulfonamide (25 mg, 45.81 μmol, 26.01% yield) as a light yellow solid.

It is noted that it cannot be excluded that be excluded that the dichloro compound 1,8-dichloro-3-(5-(difluoromethyl)-1,3,4-thiadiazol-2-yl)-N-(1-methylcyclopropyl)imidazo[1,5-a]pyridine-6-sulfonamide was also formed in this process.

**RT** 0.510 min (**method 3**); **m/z** 545.8 $(M+H)^+$ $(ESI^+)$

Preparation of Intermediate 1.11

8-chloro-3-(5-(difluoromethyl)-1,3,4-thiadiazol-2-yl)-N-(1-methylcyclopropyl)imidazo[1,5-a]pyridine-6-sulfonamide

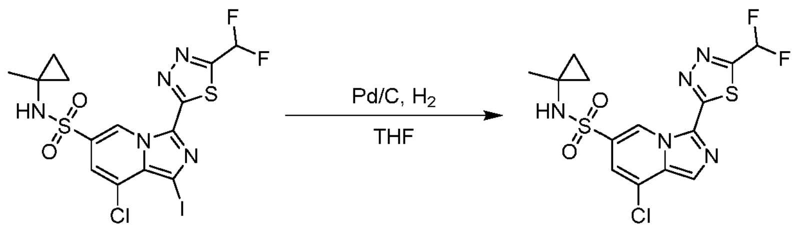


To a mixture of 8-chloro-3-(5-(difluoromethyl)-1,3,4-thiadiazol-2-yl)-1-iodo-N-(1-methylcyclopropyl)imidazo[1,5-a]pyridine-6-sulfonamide (25 mg, 45.81 μmol) in tetrahydrofuran (3 mL) was added Pd/C (5 mg, 10% purity). The reaction was degassed with $H_2$ (15 Psi) three times, then the reaction was stirred at 20°C for 3 h. The reaction mixture was filtered and the filtrate was concentrated under vacuum to give the product 8-chloro-3-(5-(difluoromethyl)-1,3,4-thiadiazol-2-yl)-N-(1-methylcyclopropyl)imidazo[1,5-a]pyridine-6-sulfonamide (20 mg, 30.96 μmol, 67.59% yield, 65% purity) as a brown solid.

It is noted that it cannot be excluded that the dichloro compound 1,8-dichloro-3-(5-(difluoromethyl)-1,3,4-thiadiazol-2-yl)-N-(1-methylcyclopropyl)imidazo[1,5-a]pyridine-6-sulfonamide was also formed in this process.

**RT** 0.468 min (**method 3**); **m/z** 420.0 $(M+H)^+$ $(ESI^+)$

Preparation of Intermediate 1.12

tert-butyl (2S,6S)-4-(3-(5-(difluoromethyl)-1,3,4-thiadiazol-2-yl)-6-(N-(1-methylcyclopropyl)sulfamoyl)imidazo[1,5-a]pyridin-8-yl)-2,6-dimethylpiperazine-1-carboxylate

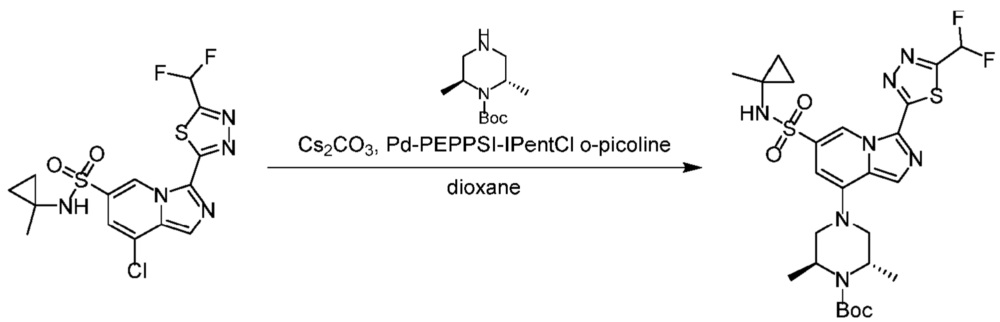


To a mixture of 8-chloro-3-(5-(difluoromethyl)-1,3,4-thiadiazol-2-yl)-N-(1-methylcyclopropyl)imidazo[1,5-a]pyridine-6-sulfonamide (30 mg, 0.0715 mmol) in dioxane (0.5mL) was added tert-butyl (2S,6S)-2,6-dimethylpiperazine-1-carboxylate (15 mg, 0.0715 mmol), $Cs_2CO_3$ (70 mg, 0.214 mmol) and Pd-PEPPSI-IPentCl o-picoline (7.0 mg, 0.00715 mmol). The reaction mixture was degassed with $N_2$ (3x) and then stirred at 98 °C for 1 h. The mixture was filtered and the

filtrate was concentrated under vacuum. The residue was purified by preparative TLC (Petroleum ether: Ethyl acetate = 1:2) to give the product tert-butyl (2S,6S)-4-(3-(5-(difluoromethyl)-1,3,4-thiadiazol-2-yl)-6-(N-(1-methylcyclopropyl)sulfamoyl)imidazo[1,5-a]pyridin-8-yl)-2,6-dimethylpiperazine-1-carboxylate (16 mg, 0.0238 mmol, 33.34% yield) as a yellow solid.

**RT** 0.573 min (**method 4**); **m/z** 598.1 (M+H$^+$) (ESI$^+$); **$^1$H NMR** ($CDCl_3$, 400 MHz): 9.74 (s, 1H), 7.83 (s, 1H), 7.08 (t, *J* = 53.6 Hz, 1H), 6.36 (s, 1H), 5.06 (s, 1H), 4.20-4.37 (m, 2H), 4.14-4.11 (m, 2H), 3.67-3.48 (m, 2H), 1.52 (s, 9H), 1.40 (s, 3H), 1.34 (d, *J* = 6.8 Hz, 6H), 0.98-0.96 (m, 2H), 0.63-0.58 (m, 2H)

Preparation of Example 1

3-(5-(difluoromethyl)-1,3,4-thiadiazol-2-yl)-8-((3S,5S)-3,5-dimethylpiperazin-1-yl)-N-(1-methylcyclopropyl)imidazo[1,5-a]pyridine-6-sulfonamide formate

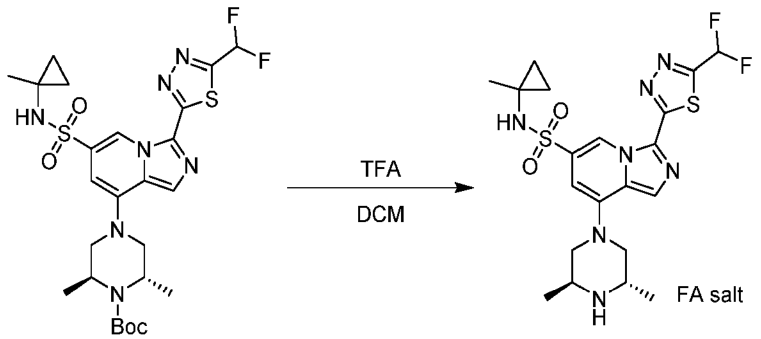


A solution of tert-butyl (2S,6S)-4-(3-(5-(difluoromethyl)-1,3,4-thiadiazol-2-yl)-6-(N-(1-methylcyclopropyl)sulfamoyl)imidazo[1,5-a]pyridin-8-yl)-2,6-dimethylpiperazine-1-carboxylate (16 mg, 0.0322 mmol) in DCM (0.5 mL) and TFA (0.1 mL) was stirred at 25 °C for 1 h. The mixture was concentrated under vacuum to give a residue, which was purified by preparative HPLC (column: Phenomenex luna C18 150*25 mm* 10 μm; mobile phase: A: 0.225% formic acid in water, B: MeCN; B%: 14%-44%, 10 min) and lyophilized directly to give the product 3-(5-(difluoromethyl)-1,3,4-thiadiazol-2-yl)-8-((3S,5S)-3,5-dimethylpiperazin-1-yl)-N-(1-methylcyclopropyl)imidazo[1,5-a]pyridine-6-sulfonamide formate (4.0 mg, 0.00725 mmol, 22.56% yield, FA salt) as a yellow solid

**RT** 0.362 min (**method 4**); **m/z** 498.2 (M+H)$^+$ (ESI$^+$); **$^1$H NMR** (DMSO-$d_6$, 400 MHz): 9.57 (s, 1H), 8.43 (s, 1H), 8.22 (s, 1H), 7.89 (s, 1H), 7.67 (t, *J* = 53.2 Hz, 1H), 6.66 (s, 1H), 3.30-3.27 (m, 2H), 3.26-3.22 (m, 2H), 3.06-3.01 (m, 2H), 1.23 (d, *J* = 6.4 Hz, 6H), 1.15 (s, 3H), 0.77-0.67 (m, 2H), 0.45-0.47 (m, 2H).

Preparation of Example 1b

3-(5-(difluoromethyl)-1,3,4-thiadiazol-2-yl)-8-((3S,5S)-3,5-dimethylpiperazin-1-yl)-N-(1-methylcyclopropyl)imidazo[1,5-a]pyridine-6-sulfonamide

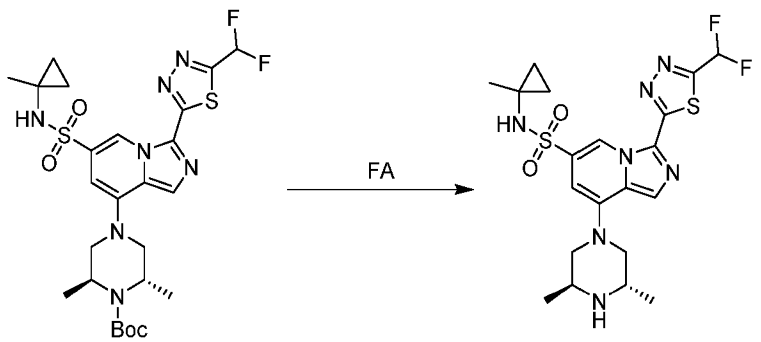


The reaction was performed twice on 29g of tert-butyl (2S,6S)-4-[3-[5-(difluoromethyl)-1,3,4-thiadiazol-2-yl]-6-[(1-methylcyclopropyl)sulfamoyl]imidazo[1,5-a]pyridin-8-yl]-2,6-dimethyl-piperazine-1-carboxylate and then, the reaction mixture were combined for the work-up.

A mixture of tert-butyl (2S,6S)-4-[3-[5-(difluoromethyl)-1,3,4-thiadiazol-2-yl]-6-[(1-methylcyclopropyl)sulfamoyl] imidazo[1,5-a]pyridin-8-yl]-2,6-dimethyl-piperazine-1-carboxylate (29.00 g, 48.5 mmol) in formic acid (290 mL, 7686 mmol,158 eq) was stirred at 20 °C for 2 h. The both reaction mixtures were combined and the formic acid was concentrated under vacuum to give a residue which was diluted with water (500 mL). The pH was adjusted to 9 with a saturated solution of $NaHCO_3$ and the product was extracted with DCM (500 mL×3). The combined organic layer was washed with brine (500 mL), dried over $Na_2SO_4$, filtered and concentrated under vacuum. The resulting yellow solid was taken up with EtOH(1.4 L) and stirred at 110 °C until complete dissolution. The resulting clear solution was stirred 12 h cooling to room temperature. The precipitate was filtered off and dried under vacuum to give a fraction A of 3-[5-(difluoromethyl)-1,3,4-thiadiazol-2-yl]-N-(1-methylcyclopropyl)-8-[(3S,5S)-3,5-dimethylpiperazin-1-yl]imidazo[1,5-a]pyridine-6-sulfonamide (33.00 g, 66.3 mmol, 68.6 % yield) as a yellow solid.

The filtrate was concentrated under vacuum to give a residue (15g) which was recrystallized in EtOH following the same procedure as depicted to get fraction A. The precipitate was filtered off and dried under vacuum to give a fraction B of 3-[5-(difluoromethyl)-1,3,4-thiadiazol-2-yl]-N-(1-methylcyclopropyl)-8-[ 3S,5S)-3,5-dimethylpiperazin-1-yl]imidazo[1,5-a]pyridine-6-sulfonamide (7.6 g, 15.3 mmol, 15.7 % yield) as a yellow solid.

Fraction A

**$^1$H NMR** (400 MHz, DMSO-$d_6$) δ ppm 9.56 (s, 1 H), 8.43 (s, 1 H), 7.86 (s, 1 H), 7.67 (t, *J* = 53.2 Hz, 1H), 6.65 (s, 1 H), 3.25 - 3.31 (m, 2 H), 3.17-3.25 (m, 2 H), 3.00 (m, 2 H), 2.15 (br s, 1 H), 1.13 - 1.22 (m, 9 H), 0.66 - 0.77 (m, 2 H), 0.41 - 0.49 (m, 2 H)

Fraction B

**$^1$H NMR** (400 MHz, DMSO-$d_6$) δ ppm 9.56 (s, 1 H), 8.43 (s, 1 H), 7.86 (s, 1 H), 7.67 (t, *J* = 53.2 Hz, 1H), 6.65 (s, 1 H), 3.25 - 3.31 (m, 2 H), 3.17-3.25 (m, 2 H), 2.95-3.07 (m, 2 H), 2.15 (br s, 1 H), 1.13 - 1.22 (m, 9 H), 0.66 - 0.77 (m, 2 H), 0.41 - 0.49 (m, 2 H)

The following Table 1 provides an overview on the compounds described in the example section:

**Table 1**

| Example No. | Structure | Name of compound |
|---|---|---|
| 1 | 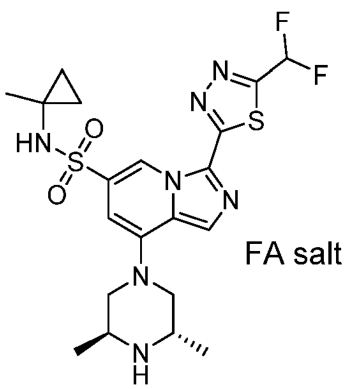 FA salt | 3-(5-(difluoromethyl)-1,3,4-thiadiazol-2-yl)-8-((3S,5S)-3,5-dimethylpiperazin-1-yl)-N-(1-methylcyclopropyl)imidazo[1,5-a]pyridine-6-sulfonamide formate |

(continued)

| Example No. | Structure | Name of compound |
|---|---|---|
| 1b | 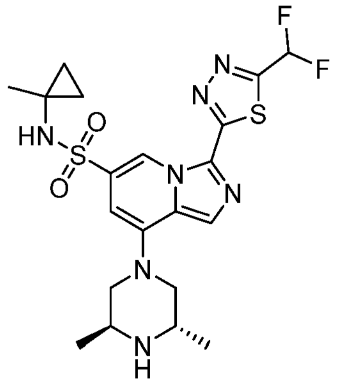 | 3-(5-(difluoromethyl)-1,3,4-thiadiazol-2-yl)-8-((3S,5S)-3,5-dimethylpiperazin-1-yl)-N-(1-methylcyclopropyl)imidazo[1,5-a]pyridine-6-sulfonamide |

**Biological evaluation of the exemplary compounds**

The compound of formula (I) was tested in selected biological and/or physicochemical assays one or more times. When tested more than once, data are reported as either average values or as median values, wherein the average value, also referred to as the arithmetic mean value, represents the sum of the values obtained divided by the number of times tested, and the median value represents the middle number of the group of values when ranked in ascending or descending order. If the number of values in the data set is odd, the median value is the middle value. If the number of values in the data set is even, the median is the arithmetic mean of the two middle values. The in vitro pharmacological, pharmacokinetic and physicochemical properties of the compounds can be determined according to the following assays and methods.

PARG protein expression and purification

A codon optimized gene encoding human PARG (448-976 [H446G, L447S, L473S, N479S, S802A, R811K, M841I, S858P, I916T, T924D, D927K, C963S, A967T]) was synthesized by Genscript, and cloned into pET15b (NcoI/BamHI) with an N-terminal, Thrombin protease cleavable 6His-TwinStrep tag. Expression of the protein in *E. coli* BL21 (DE3) was induced by addition of 0.2 mM IPTG to a shake flask culture grown to OD600=0.8 at 37°C. Growth was allowed to continue at 30°C for a further 20 hours before harvesting by centrifugation and storage of the cell pellet at -80°C.

Protein was purified by IMAC and SEC: frozen cell pellets (typically 40 g wet weight) were resuspended by homogenization in 5 volumes buffer A (25 mM Tris/HCl pH 8.0, 200 mM NaCl, 2 mM DTT), supplemented with 1 mg of DNase I from bovine pancreas (Sigma-Aldrich) and protease inhibitors (Roche Complete™ EDTA-free protease inhibitor tablet), and lysed by passage through a Constant Systems BasicZ homogenizer. The lysate was clarified by centrifugation for 60 minutes at 25,000 g, 4°C, and the lysate supernatant was loaded onto 5 ml StrepTrap HP (Cytiva) pre-equilibrated with buffer A. The column was washed with buffer A (~10 CV), then buffer B containing 1M KCl (~5 CV), and then the protein was eluted with buffer A containing 2.5 mM d-Desthiobiotin. Pooled fractions containing 6HisTwinStrep-TEV-hPARG were incubated with TEV protease overnight at 4°C. hPARG was separated from uncleaved material and Thrombin protease through gel filtration with Superdex75 sizing column (GE Healthcare) pre-equilibrated with SEC buffer (15 mM Tris/HCl pH 8.5, 100 mM NaCl, 2 mM DTT). Pooled fractions containing pure hPARG were concentrated using a 10 k MWCO spin concentrator (VivaSpin) to 10 mg/mL, and then either used immediately for crystallisation or snap-frozen in liquid nitrogen for storage at -80°C.

PARG enzymatic $IC_{50}$ assay

PARG enzyme as incubated with compound or vehicle (DMSO) for 15 minutes or 2 hours in a 384 well plate. After adding the PARG substrate ADP-ribose-pNP, the plate was read for absorbance intensity at 405 nm. The vehicle (DMSO) with high absorbance intensity represents no inhibition of enzymatic reaction while the low control (no enzyme) with low absorbance intensity represents full inhibition of enzymatic reaction.

Materials:

hPARG: Peak Protein, 30 nM
Substrate: ADP-pNP, 800 μM, Jena Bioscience catalog # NU-955
Reaction time: 60 minutes
Assay buffer: 50 mM Tris-HCl pH 8.0, 100 mM NaCl, 2 mM DTT
Temperature: 30 °C
Total volume: 30 μL

Controls:

- 0% inhibition control: DMSO
- 100% inhibition control: No enzyme

The protocol that was used for enzyme reaction and detection is as follows:

1. Transfer 100 nL of the final concentration of test compounds or vehicle (DMSO) to the appropriate wells of a microtiter plate.
2. Centrifuge the plate at 1000 rpm for 1 minute.
3. Transfer 14.6 μL of 2x final concentration of enzyme in assay buffer or assay buffer alone to the appropriate wells.
4. Centrifuge the plate at 1000 rpm for 1 minute.
5. Incubate the plate at room temperature for 15 minutes or 2 hours.
6. Transfer 15.4 μL of 2x substrate in assay buffer to all the test wells.
7. Centrifuge the plate at 1000 rpm for 1 minute.
8. Read the plate on a plate reader (e.g., Spark Tecan).

The Absorbance $IC_{50}$ value of compounds of Formula (I) in Example 1 is provided in Table 2 below.

<u>Cellular PAR chain assay</u>

The ability of compounds to inhibit PARG in response to DNA damage, was assessed with U2OS cells pretreated with the compounds for 1 hour, following a 1-hour treatment with or without the DNA alkylating agent temozolomide (TMZ). The cells were harvested and fixed in 70% ethanol, rehydrated with glucose and EDTA in PBS and subsequently blocked for 1 hour with PBS 1% BSA and 0.01% Tween-20 (PBT). The cells were incubated for 2 hours at room temperature with a mouse monoclonal antibody against poly (ADP) ribose (PAR) polymer. The cells were washed and incubated with an anti-mouse Alexa-488 conjugated secondary antibody for 1 hour at room temperature. Propidium iodide staining was used to determine DNA content in the cells (staining at 4°C overnight). The fluorescence intensity of the cells was assessed by flow cytometry (Cytoflex from Beckmann) and the percentage of PAR chain positive cells (gated in relation to TMZ+DMSO treated control) was determined. PAR chain positive cells % were fit against the concentration of the compound using a 4 parameter log-logistic function, generating PAR chain $EC_{50}$ values:

$$f(x) = c + \frac{d - c}{1 + exp^{b(\log(x) - \log(e))}}$$

The PAR chain $EC_{50}$ value for compounds of Formula (I) in Example 1 is provided in Table 2 below.

<u>Cellular Viability Assay</u>

NCI-H460 and MDA-MB-436 as PARG-inhibition sensitive cell lines, and U2OS as PARG-inhibition insensitive cell line, were plated at 1000 cells/well, 5000 cells/well and 2000 cells/well, respectively, in 96-well white plates with clear flat bottom. After 24 hours, the compounds were added with the Tecan digital dispenser (D300e), in duplicates. The outer wells of the plate were excluded. After 96 hours of incubation, 150 μl of the growth medium were removed and 50 μl of Cell

Titer-Glo (Promega) were added per well. Following an incubation of 10 minutes, luminescence was read using a plate reader (Tecan) for NCI-H460 and U2OS cell lines, and a 2104 EnVision plate reader for MDA-MB-436. Averaged values of the samples were normalized to DMSO treated control samples. Curves were fit as % of the control vs. log of the compound concentration using a 4 parameter log-logistic function:

$$f(x) = c + \frac{d - c}{1 + exp^{b(\log(x) - \log(e))}}$$

The PARGi (NCI-H460, MDA-MB-436 and U2OS) cellular viability $EC_{50}$ values for compounds of Formula (I) in Examples 1 and 1b are provided in Table 2 below.

**Table 2:** Inhibition of PARG and cellular activity of compounds according to the present invention.

The $IC_{50}$ (inhibitory concentration at 50% of maximal effect) values are indicated in µM, empty space means that the corresponding compounds have not been tested in the respective assay.

① Example number
③ $IC_{50}$ in µM determined in PARG enzymatic assay (PARG protein and 15 mn incubation) described under PARG enzymatic $IC_{50}$ assay
④ $IC_{50}$ in µM determined in PARG enzymatic assay (PARG protein and 2 hours incubation) described under PARG enzymatic $IC_{50}$ assay
⑤ $EC_{50}$ in µM determined in cellular assay as described under Cellular PAR chain assay (conditions with treatment of TMZ).
⑥ $EC_{50}$ in µM determined in cellular assay as described under Cellular PAR chain assay (conditions without treatment of TMZ).
⑦ $EC_{50}$ in µM determined in NCIH-460 cells as described under Cellular viability assay.
⑧ $EC_{50}$ in µM determined in U2OS cells as described under Cellular viability assay.
⑨ $EC_{50}$ in µM determined in MDA-MB-436 cells as described under Cellular viability assay
⑩ Kinetic solubility pH 7.4 (µM)

**Table 2.**

| ① | ③ | ④ | ⑤ | ⑥ | ⑦ | ⑧ | ⑨ | ⑩ |
|---|---|---|---|---|---|---|---|---|
| **1** | | 0.011 | <0.003 | <0.003 | <0.019 | 11.9 | 0.017 | 152 |
| **1b fraction A** | | | | | 0.021 | >2.00 | | |
| **1b fraction B** | | | | | 0.022 | >2.00 | | |

## Further assays

Kinetic solubility assay

The Kinetic solubility assay employs the shake flask method followed by HPLC-UV analysis. For exemplary compounds, the kinetic solubility was measured according to the following protocol:

1. Samples were weighed and dissolved in 100% DMSO to make a stock solution of 10 mM. About 100 µL of stock solution is needed to cover this assay.
2. Test compounds and controls (10 mM in DMSO, 10 µL/tube) were added into the buffer (490 µL/well) which were placed in a Mini-UniPrep filter. The buffer was prepared as the customer's requirement.
3. The kinetic solubility samples were vortexed for 2 minutes.
4. The solubility solutions were shaked in an orbital shaker for 24 hr at room temperature.
5. 200 µL of each solubility solution were transferd into a 96-deep well for analysis when the samples were directly filtered by the syringeless filter device.
6. The test compound concentration of the filtrate were determined using HPLC-UV.
7. Three UV standard solutions were injected into HPLC from low to high concentrations, followed by testing of the K.S. supernatant. Testing samples were injected in duplicate.

Bidirectional permeability in Caco2

The bidirectional permeability in Caco-2 cells assay was performed for the exemplary compounds of formula (I) according to the following protocol:

1. Caco-2 cells purchased from ATCC were seeded onto polyethylene membranes (PET) in 96-well BD insert plates at 1 x 105 cells/$cm^2$, and refreshed medium every 4~5 days until to the $21^{st}$ to $28^{th}$ day for confluent cell monolayer formation.
2. The integrity of the monolayer was verified by performing Lucifer yellow rejection assay.
3. The quality of the monolayer was verified by measuring the unidirectional (A→B) permeability of fenoterol/nadolol (low permeability marker), propranolol/metopronolol (high permeability marker) and bi-directional permeability of digoxin (a P-glycoprotein substrate marker) in duplicate wells.
4. Standard assay conditions for test compounds:

- test concentration: 2 μM (DMSO≤1%);
- replicates: n=2;
- directions: bi-directional transport including A→B and B→A;
- incubation time: single time point, 2hours;
- transport buffer: HBSS containing 10 mM HEPES, pH7.40±0.05;
- incubation condition: 37±1°C, 5% $CO_2$, relatively saturated humidity.

5. Dosing solution were spiked and mixed with transport buffer and stop solution (containing an appropriate internal standard (IS)) as T0 sample.
6. At the end of incubation, sample solutions from both donor and receiver wells were mixed with stop solution immediately.
7. All samples including T0 samples, donor samples and receiver samples were analyzed using LC/MS/MS. Concentrations of test compound were expressed as peak area ratio of analytes versus IS without a standard curve.

Liver Microsomes Stability Assay

1. Materials

1.1 Liver microsomes
Animal or human liver microsomes were purchased from Xenotech or Corning and stored in a freezer (lower than -60°C) before use.
1.2 β-nicotinamide adenine dinucleotide phosphate reduced form, tetrasodium salt, Vendor: Chem-Impex International, Cat.No. 00616
1.3 Control compounds: Testosterone, diclofenac and propafenone.

2. Preparation of Working Solution

Stock Solution: 10 mM test compound in DMSO.
Working solution: 100 μM test or control compounds in 100% acetonitrile (concentration of organic solvent: 1% (v/v) DMSO and 99% (v/v) acetonitrile)

3. Assay Procedure

A total of two sample plates with 96-well format were prepared for incubation, labeled as 'Incubation' T60 and 'Incubation' NCF60. Empty 'Incubation' T60 and NCF60 plates were pre-warmed for 10 min minutes. Liver microsomes were diluted to 0.56 mg/mL in 100 mM phosphate buffer. Microsome working solutions (0.56 mg/mL) were transferred (445 uL) into pre-warmed 'Incubation' T60 and NCF60 plates, followed by incubation for 10 min at 37°C with constant shaking.
Liver microsomes (54 μL) were transferred to a Blank60 plate, followed by the addition of 6 μL NAPDH cofactor and 180 μL stop solution (acetonitrile containing internal standards) into each well.
An aliquot (5 μL) of compound working solution (100 μM) was added into the 'incubation' plates (T60 and NCF60)

containing microsomes and mixed 3 times thoroughly.

For the 'Incubation' NCF60 plate, 50 uL of buffer was added and mixed 3 times thoroughly. The plates were incubated at 37°C for 60 min while shaking. samples were mixed once and 60 μL was transferred from the NCF60 incubation plate to the stop plate containing stop solution after the 60-min incubation.

Stop solution (180 μL) and NAPDH cofactor (6 μL) were added to 'Quenching' plate T0. Plates were chilled to prevent evaporation.

For the 'Incubation' T60 plate: mixed 3 times thoroughly, and immediately removed 54 μL mixture for the 0-min time point to stop plate ('Quenching' plate T0). NAPDH cofactor (44 μL) was added to the 'Incubation' T60 plate. The plate was incubated at 37°C for 60 min while shaking. At 5, 15, 30, 45, and 60 min, 180 μL stop solution was added to the 'Quenching' plates, samples were mixed once, and 60 μL was serially transferred from 'Incubation' T60 plate per time point.

Consequently, for the wells containing the test or control compounds, the final concentration was 1 μM for test compounds, testosterone, diclofenac and propafenone, 0.5 mg/mL for animal or human liver microsomes, 0.01% (v/v) for DMSO and 0.99% (v/v) for acetonitrile.

All sampling plates were shaken for 10 min, then centrifuged at 3220 ×g for 20 minutes at 4°C. Supernatant (80 μL) was transferred into 240 μL HPLC water, and mixed by plate shaker for 10 min. Each bioanalysis plate was sealed and shaken for 10 minutes prior to LC-MS/MS analysis.

4. Bioanalytical Analysis

Concentrations of test conpounds and positive controls, testosterone, diclofenac and propafenone in the samples were determined by using a liquid chromatography-tandem mass spectrometry (LC-MS/MS) method.

5. Data Calculation

In the determination of the in vitro elimination constant, ke, of test compound and control compounds, the peak area ratios (PAR) of analyte/internal standard were used to calculate the percentage of remaining (%Remaining) with the following equation:

$$\%\text{Remaining} = \frac{\text{PAR of analyte to internal standard at each time point}}{\text{PAR of analyte to internal standard at } T = 0} \times 100$$

$$C_t = C_0 \bullet e^{-k_e \bullet t}$$

when $C_t = \frac{1}{2} C_0$ ,

$$T_{1/2} = \frac{\text{Ln}2}{k_e} = \frac{0.693}{k_e}$$

$$CL_{int}(\text{mic}) = 0.693 / T_{1/2} / \text{mg microsome protein per mL}$$

$$CL_{int}(\text{liver}) = CL_{int}(\text{mic}) \times \text{mg microsomal protein/g liver weight} \times \text{g liver weight/kg body weight}$$

According to the well-stirred model, hepatic intrinsic clearance and hepatic clearance can be calculated by the following formula.

$$CL(\text{liver}) = (\text{CLint(liver)} \times \text{fu} \times \text{Qh}) / (\text{CLint(liver)} \times \text{fu} + \text{Qh})$$

The default value of $f_u$ (the fraction unbound in blood) is assumed as 1.

The parameters in equations are listed in following table.

| Species | Liver Weight (g/kg Body Weight) [1-2] | Hepatic Blood Flow ($Q_h$) (mL/min/kg) [1-2] | Microsomal Protein (mg/g liver weight) |
|---|---|---|---|
| Mouse | 88 | 90.0 | 45 |
| Rat | 40 | 55.2 | |
| Dog | 32 | 30.9 | |
| Monkey | 30 | 43.6 | |
| Human | 20 | 20.7 | |

When the %Remaining value at the maximal incubation time, which was 60 min in this study, was higher than 75%, it is considered to be within the acceptable experimental variation, i.e., CV =25%. Therefore, a corresponding T1/2 value of >145 min is reported. Consequently, the corresponding CLint(mic)value is reported as <9.6 μL/min/mg protein.

6. References

[1] Brian Davies and Tim Morris, Physiological Parameters in Laboratory Animals and Human. Pharmaceutical Research, Vol. 10 No.7, 1993

[2] Journal of Pharmacology and Experimental Therapeutics, 1997, 283(1): 46-58


Hepatocyte Metabolic Stability

1. Materials

1.1 Hepatocyte

Animal or human hepatocytes were purchased from BioreclamationIVT or RILD.

1.2 Control compounds:

7-Ethoxycoumarin and 7-Hydroxycoumarin

2. Preparation of Working Solution

Stock Solution: 10 mM test compound and 30 mM control compound in DMSO.

Working solution: 100 μM test compound or 300 μM control compounds in 100% acetonitrile (Concentration of organic solvent: 1% (v/v) DMSO and 99% (v/v) acetonitrile)

3. Assay Procedure

Cryopreserved hepatocytes were thawed, isolated, and suspended in Williams' Medium E, then diluted with pre-incubated Williams' Medium E to a final concentration of 0.510×106 cells/mL. One hundred and ninety-eight (198) μL of cells suspension (0.510×106 cells/mL) were added into appropriate wells. The incubation plate was pre-incubated in a 37.0°C incubator for about 10 minutes. Then 2 μL of test compound and positive controls were added into plate except for the blank plate. Incubate all plates at 37.0°C in a 95.0% humidified incubator at 5.0% CO2 to start the reactions with constant shaking.

For the T0 plate, a corresponding quenching plate was prepared by adding 125 μL/well of acetonitrile containing 200 ng/mL tolbutamide and 200 ng/mL labetalol as internal standards (stop solution), and 25 μL/well of the incubation sample were transferred to this plate after shaking for 1 minute to ensure homogeneity.

At each time-point, the corresponding plate was removed from the incubator, and 25 μL/well of the corresponding sample was transferred to its corresponding quenching plate containing 125 μL/well of stop solution. Medium control (MC) plates (T0-MC and T90-MC) were prepared by adding everything except for Williams' Medium E at the corresponding time-points.

The plates were then sealed and shaken for 10 minutes prior to centrifugation at 4000 rpm and 4°C for 20 minutes.

80 μL/well of the resulting supernatant were diluted with 240 μL/well of pure water and sealed and shaken for 10 minutes prior to LC-MS/MS analysis.

4. Bioanalytical Analysis

Concentrations of test compounds and positive controls, 7-Ethoxycoumarin and 7-Hydroxycoumarin in the samples were determined by using a liquid chromatography-tandem mass spectrometry (LC-MS/MS) method.

5. Data Calculation

In the determination of the in vitro elimination constant, ke, of test compound and control compounds, the peak area ratios (PAR) of analyte/internal standard were used to calculate the percentage of remaining (%Remaining) with the following equation:

$$\%\text{Remaining} = \frac{\text{PAR of analyte to internal standard at each time point}}{\text{PAR of analyte to internal standard at } T = 0} \times 100$$

$$C_t = C_0 \bullet e^{-k_e \bullet t}$$

when $C_t = \frac{1}{2} C_0$ ,

$$T_{1/2} = \frac{\text{Ln}2}{k_e} = \frac{0.693}{k_e}$$

$$CL_{int\ (hep)} = k\ /\ \text{million cells per mL}$$

$$CL_{int\ (liver)} = CL_{int\ (hep)} \times \text{liver weight (g/kg body weight)} \times \text{hepatocellularity}$$

According to the well-stirred model, hepatic intrinsic clearance and hepatic clearance can be calculated by the following formula.

$$CL_{(liver)} = (CL_{int(liver)} \times f_u \times Q_h) / (CL_{int(liver)} \times f_u + Q_h)$$

The default value of $f_u$ (the fraction unbound in blood) is assumed as 1. The parameters in equations are listed in following table.

| Species | Liver Weight (g/kg Body Weight) [2-3] | Liver Blood Flow ($Q_h$) (mL/min/kg) [2-3] | Hepatocellularity (of cells/g liver) [1] |
|---|---|---|---|
| Mouse | 88 | 90.0 | $135\times10^6$ |
| Rat | 40 | 55.2 | $117\times10^6$ |
| Dog | 32 | 30.9 | $215\times10^6$ |
| Monkey | 30 | 43.6 | $120\times10^6$ |
| Human | 20 | 20.7 | $139\times10^6$ |

When the %Remaining value at the maximal incubation time, which was 90 min in this study, was higher than 75%, it is considered to be within the acceptable experimental variation, i.e., CV =25%. Therefore, a corresponding T1/2 value of >216.8 min is reported. Consequently, the corresponding CLint(hep) (μL/min/106 cells) is reported as <7.5.

6. References

[1] Anna-Karin Sohlenius-Sternbeck. Determination of the hepatocellularity number for human, dog, rabbit, rat and mouse livers from protein concentration measurements. Toxicology in Vitro, Vol. 20 No.8, 2006
[2] Brian Davies and Tim Morris, Physiological Parameters in Laboratory Animals and Human. Pharmaceutical Research, Vol. 10 No.7, 1993
[3] Obach R S, Baxter J G, Liston T E, et al. The prediction of human pharmacokinetic parameters from preclinical and in vitro metabolism data [J]. Journal of Pharmacology and Experimental Therapeutics, 1997, 283(1): 46

Cell-derived xenograft (CDX) study

**Materials:**

Animals

Species: *Mus musculus*
Strain: BALB/c nude
Age: 6-9 weeks
Sex: Female
Body weight: 17-20 g
Animal supplier: Beijing Vital River Laboratory Animal Technology Co., Ltd Certificate No: 20230209Abzz0619000498

Cells
The MDA-MB-436 tumor cells (ATCC, cat #HTB-130) were maintained *in vitro* as a monolayer culture in L-15 medium supplemented with 10% fetal bovine serum, 10 μg/mL Insulin, 16 μg/mL glutathione and 1% Antibiotic-Antimycotic at 37°C in an atmosphere of 0% $CO_2$ in air. The cells growing in an exponential growth phase were harvested and counted for tumor inoculation.

**In vivo CDX study:**

BALB/c nude female mice were inoculated subcutaneously at the right flank with MDA-MB-436 tumor cells (10 x $10^6$/mouse) in 0.2 mL of PBS with Matrigel (1:1) for tumor development. The animals were randomized and the treatment was started on day 25 after tumor inoculation when the average tumor volume reached approximately 126 $mm^3$. Each group consisted of 10 tumor-bearing mice. The compound of formula (I) was administrated to the mice according to the predetermined regimen as shown in the experimental design table 3

**Table 3.** Description of experimental design.

| Group | N[a] | Treatment | Dose (mg/kg) | Dose Volume (μL/g)[b] | Dosing Route | Schedule[c] |
|---|---|---|---|---|---|---|
| 1 | 10 | Vehicle | -- | 10 | PO | QD x 28 days |
| 2 | 10 | Example 1 | 100 | 10 | PO | QD x 28 days |

Note:
a. N: number of animals per group;
b. Dosing volume: adjust dosing volume based on body weight 10 μL/g;
c. The experiment duration was 28 days.

Tumor sizes were measured twice weekly in two dimensions using a caliper, and the volumes were expressed in $mm^3$. The tumor sizes were then used for the calculations of TGI values.

TGI was calculated for each group using the formula: TGI (%) = [1-(Ti-T0)/ (Vi-V0)] × 100; Ti is the average tumor volume of a treatment group on a given day, T0 is the average tumor volume of the treatment group on the first day of treatment, Vi is the average tumor volume of the vehicle control group on the same day with Ti, and V0 is the average tumor volume of the vehicle group on the first day of treatment.

A one-way ANOVA was performed to compare the tumor volume among groups.

The compound of formula (I) (i.e. Example 1 - its formate salt form) given at 100 mg/kg resulted in a significant and robust anti-tumor response, with tumor regression seen in all mice (Table 4, FIG. 1).

**Tumor Growth Inhibition Analysis**

**Table 4.** Tumor growth inhibition calculation for Example 1 in the MDA-MB-436 xenograft model calculated based on tumor volume measurements on day 28.

| Treatment | N | Tumor volume ($mm^3$)[a] on day 28 | TGI[b] (%) | *p* value[c] |
|---|---|---|---|---|
| Vehicle | 10 | 668 ± 90 | - | - |
| Example 1, 100 mg/kg | 10 | 27 ± 9 | 118.20 | <0.001 |

Note:
a. Mean ± SEM;
b. TGI (%) = $[1-(T_{28}-T_0)/(V_{28}-V_0)] \times 100$;
c. *p* value is calculated based on tumor volume.

## Claims

1. A compound of formula (I):

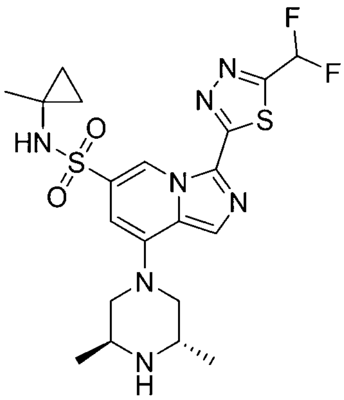

(I)

or a tautomer, pharmaceutically acceptable solvate, pharmaceutically acceptable crystal form, or pharmaceutically acceptable salt thereof.

2. The compound of claim 1, wherein the compound is a compound of formula (I) or a pharmaceutically acceptable salt thereof.

3. The compound of claim 2, in a non-salt form.

4. The compound of claim 2, which is a formate salt of the compound of formula (I).

5. A pharmaceutical composition comprising the compound of any one of claims 1 to 4 or a pharmaceutically acceptable salt, hydrate or solvate thereof, and a pharmaceutically acceptable carrier.

6. The compound of any one of claims 1 to 4 or a pharmaceutically acceptable salt, hydrate or solvate thereof, or a pharmaceutical composition of claim 5, for use in therapy.

7. The compound for use or the pharmaceutical composition for use of claim 6, for use in a method of treating a disease or

disorder in which PARG activity is implicated.

**8.** The compound for use or the pharmaceutical composition for use of claim 7, for use in a method of treating a proliferative disorder.

**9.** The compound for use or the pharmaceutical composition for use of claim 8, wherein the proliferative disorder is selected from malignant neoplasms and tumours, cancers, leukemias, psoriasis, bone diseases, fibroproliferative disorders, and atherosclerosis.

**10.** The compound for use or the pharmaceutical composition for use of claim 8, wherein the proliferative disorder is cancer.

**11.** The compound for use or the pharmaceutical composition for use of claim 10, wherein cancer is selected from lung, colon, breast, ovarian, prostate, liver, pancreas, brain, and skin cancer.

**Patentansprüche**

**1.** Verbindung der Formel (I):

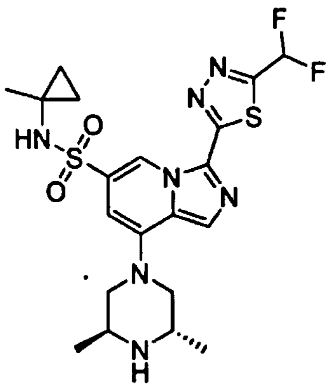

(I)

oder ein Tautomer, ein pharmazeutisch verträgliches Solvat, eine pharmazeutisch verträgliche Kristallform oder ein pharmazeutisch verträgliches Salz davon.

**2.** Verbindung nach Anspruch 1, wobei die Verbindung eine Verbindung der Formel (I) oder eine pharmazeutisch verträgliches Salz davon ist.

**3.** Verbindung nach Anspruch 2, in einer Nicht-Salz-Form.

**4.** Verbindung nach Anspruch 2, die ein Formatsalz der Verbindung der Formel (I) ist.

**5.** Arzneimittel, das die Verbindung nach einem der Ansprüche 1 bis 4 oder ein pharmazeutisch verträgliches Salz, Hydrat oder Solvat davon und einen pharmazeutisch verträglichen Träger umfasst.

**6.** Verbindung nach einem der Ansprüche 1 bis 4 oder pharmazeutisch verträgliches Salz, Hydrat oder Solvat davon oder Arzneimittel nach Anspruch 5 zur Verwendung in der Therapie.

**7.** Verbindung zur Verwendung oder Arzneimittel zur Verwendung nach Anspruch 6, zur Verwendung in einem Verfahren zur Behandlung einer Erkrankung oder Störung, in der PARG-Aktivität beteiligt ist.

**8.** Verbindung zur Verwendung oder Arzneimittel zur Verwendung nach Anspruch 7, zur Verwendung in einem Verfahren zur Behandlung einer proliferativen Störung.

9. Verbindung zur Verwendung oder Arzneimittel zur Verwendung nach Anspruch 8, wobei die proliferative Störung ausgewählt ist aus bösartigen Neoplasmen und Tumoren, Krebsarten, Leukämien, Psoriasis, Knochenerkrankungen, fibroproliferativen Störungen und Artherosklerose.

10. Verbindung zur Verwendung oder Arzneimittel zur Verwendung nach Anspruch 8, wobei die proliferative Störung Krebs ist.

11. Verbindung zur Verwendung oder Arzneimittel zur Verwendung nach Anspruch 10, wobei der Krebs ausgewählt ist aus Lungen-, Darm-, Brust-, Eierstock-, Prostata-, Leber-, Pankreaskrebs, Hirntumor und Hautkrebs.

## Revendications

1. Composé de la formule (I) :

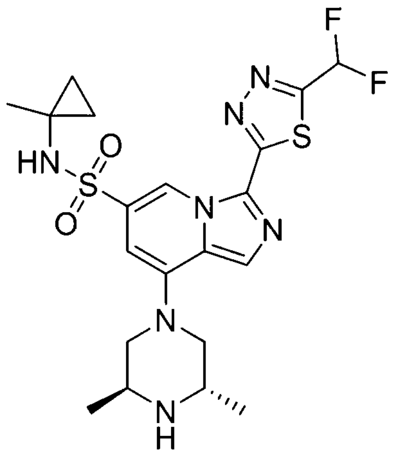

(I)

ou un tautomère, un solvate pharmaceutiquement acceptable, une forme cristalline pharmaceutiquement acceptable ou un sel pharmaceutiquement acceptable de celui-ci.

2. Composé selon la revendication 1, dans lequel le composé est un composé de la formule (I) ou un sel pharmaceutiquement acceptable de celui-ci.

3. Composé selon la revendication 2, sous une forme non saline.

4. Composé selon la revendication 2, qui est un sel de formiate du composé de la formule (I).

5. Composition pharmaceutique comprenant le composé selon l'une quelconque des revendications 1 à 4 ou l'un de ses sels, hydrates ou solvates pharmaceutiquement acceptables, et un support pharmaceutiquement acceptable.

6. Composé selon l'une quelconque des revendications 1 à 4 ou l'un de ses sels, hydrates ou solvates pharmaceutiquement acceptables, ou composition pharmaceutique selon la revendication 5, pour une utilisation en thérapie.

7. Composé à utiliser ou composition pharmaceutique à utiliser selon la revendication 6, pour une utilisation dans un procédé de traitement d'une maladie ou d'un trouble dans lequel l'activité PARG est impliquée.

8. Composé à utiliser ou composition pharmaceutique à utiliser selon la revendication 7, pour une utilisation dans un procédé de traitement d'un trouble prolifératif.

9. Composé à utiliser ou composition pharmaceutique à utiliser selon la revendication 8, dans lequel le trouble prolifératif est choisi parmi les néoplasmes et les tumeurs malignes, les cancers, les leucémies, le psoriasis, les maladies osseuses, les troubles fibroprolifératifs et l'athérosclérose.

**10.** Composé à utiliser ou composition pharmaceutique à utiliser selon la revendication 8, dans lequel le trouble prolifératif est un cancer.

**11.** Composé à utiliser ou composition pharmaceutique à utiliser selon la revendication 10, dans lequel le cancer est choisi parmi les cancers du poumon, du côlon, du sein, des ovaires, de la prostate, du foie, du pancréas, du cerveau et de la peau.

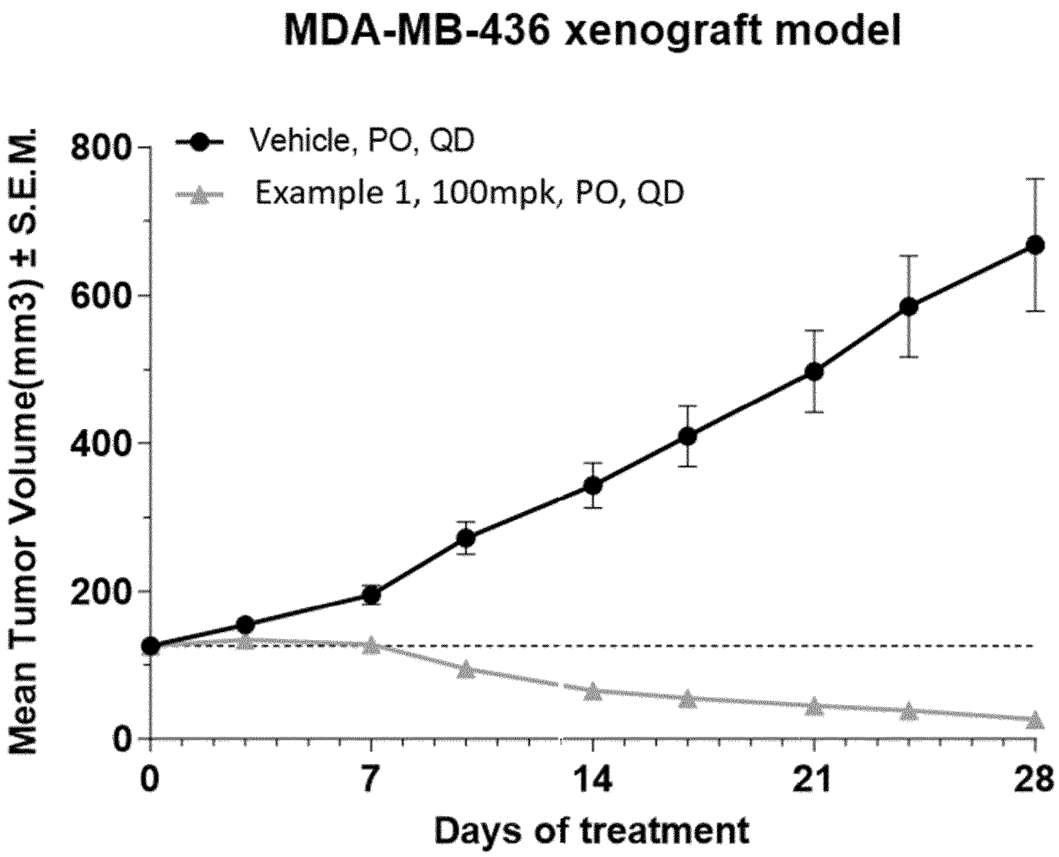
MDA-MB-436 xenograft model
Vehicle, PO, QD
Example 1, 100mpk, PO, QD
Mean Tumor Volume(mm3) ± S.E.M.
800
600
400
200
0
0
7
14
21
28
Days of treatment

Figure 1.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016092326 A
- WO 2016097749 A
- WO 2021055744 A
- US 2019233411 A
- WO 2009050183 A
- WO 0194341 A
- WO 0047212 A
- WO 9722596 A
- WO 9730035 A
- WO 9732856 A
- WO 9813354 A
- WO 9902166 A
- WO 0040529 A
- WO 0041669 A
- WO 0192224 A
- WO 0204434 A
- WO 0208213 A


### Non-patent literature cited in the description


- **JEMAL et al.** *J. Natl. Cancer Inst.*, 2017, vol. 109, 1975
- **HALAZONETIS et al.** *Science*, 2008, vol. 319, 1352
- **NEGRINI et al.** *Nat. Rev. Mol. Cell Biol.*, 2010, vol. 11, 220
- **ZEMAN** ; **CIMPRICH**. *Nat. Cell Biol.*, 2013, vol. 16 (2)
- **COSTANTINO et al.** *Science*, 2014, vol. 343, 88
- **SCULLY et al.** *Curr. Opin. Genet. Dev.*, 2021, vol. 71, 154
- **COHEN** ; **CHANG**. *Nat. Chem. Biol.*, 2018, vol. 14, 236
- **O'SULLIVAN et al.** *Nat. Commun.*, 2019, vol. 10, 1182
- **FONTANA et al.** *Elife*, 2017
- **RACK et al.** *Genes Dev.*, 2020, vol. 34, 263
- **BRYANT et al.** *Nature*, 2005, vol. 434, 913
- **FARMER et al.** *Nature*, 2005, vol. 434, 917
- **PILLAY et al.** *Cancer Cell.*, 2019, vol. 35, 519
- **FUJIHARA et al.** *Curr. Cancer Drug Targets*, 2009, vol. 9, 953
- **GOGOLA et al.** *Cancer Cell*, 2018, vol. 33, 1078
- **HOUL et al.** *Nat Commun.*, 2019, vol. 10, 5654
- **JAMES et al.** *ACS Chem. Biol.*, 2016, vol. 11, 3179
- **WASZKOWYCZ et al.** *J. Med. Chem.*, 2018, vol. 61, 10767
- **ATZRODT J et al.** *Bioorg Med Chem*, 2012, vol. 20 (18), 5658-5667
- **WILLIAM JS et al.** *Journal of Labelled Compounds and Radiopharmaceuticals*, 2010, vol. 53 (11-12), 635-644
- **MODVIG A et al.** *J Org Chem*, 2014, vol. 79, 5861-5868
- *CHEMICAL ABSTRACTS*, 70142-34-6
- Remington: The Science and Practice of Pharmacy. Pharmaceutical Press
- *J. Med. Chem.*, 2004, vol. 47, 6658-6661
- **STERN et al.** *Critical reviews in oncology/haematology*, 2005, vol. 54, 1 1-29
- **BRIAN DAVIES** ; **TIM MORRIS**. Physiological Parameters in Laboratory Animals and Human. *Pharmaceutical Research*, 1993, vol. 10 (7)
- *Journal of Pharmacology and Experimental Therapeutics*, 1997, vol. 283 (1), 46-58
- **ANNA-KARIN** ; **SOHLENIUS-STERNBECK**. Determination of the hepatocellularity number for human, dog, rabbit, rat and mouse livers from protein concentration measurements. *Toxicology in Vitro*, 2006, vol. 20 (8)
- **OBACH R S** ; **BAXTER J G** ; **LISTON T E et al.** The prediction of human pharmacokinetic parameters from preclinical and in vitro metabolism data [J. *Journal of Pharmacology and Experimental Therapeutics*, 1997, vol. 283 (1), 46